# EUROPEAN PATENT SPECIFICATION

(11) **EP 3 893 984 B1**
(45) Date of publication and mention of the grant of the patent: **31.01.2024**
(21) Application number: 19832292.7
(22) Date of filing: 09.12.2019
(51) Int. Cl.: A61M 25/06, A61M 39/06

(54) **CATHETER DEVICES WITH BLOOD CONTROL SYSTEMS AND RELATED METHODS**
KATHETERVORRICHTUNGEN MIT BLUTKONTROLLSYSTEMEN UND ZUGEHÖRIGE VERFAHREN
DISPOSITIFS DE CATHÉTER AVEC SYSTÈMES DE CONTRÔLE DU SANG ET PROCÉDÉS ASSOCIÉS

(30) Priority: 10.12.2018 US 201862777582 P
(43) Date of publication of application: 20.10.2021
(73) Proprietor: B. Braun Melsungen AG, 34212 Melsungen (DE)
(72) Inventor: NEOH, Boon Ping, Penang, 10810 (MY); NG, Jarryd Keng Gene, Penang, 10810 (MY); PHANG, Chee Mun, Penang, 10810 (MY); CHNG, Hang Khiang, Penang, 10810 (MY)
(74) Representative: Kinkeldey, Daniela
(86) International application number: PCT/EP2019/084247
(87) International publication number: WO 2020/120404

(56) References cited:
- EP-A1- 3 058 977
- US-A1- 2006 155 245
- US-A1- 2012 271 235
- US-A1- 2012 277 680

## Description

### FIELD OF ART

The disclosed invention generally relates to needle devices and intravenous (IV) infusion devices, including IV catheters. In particular, IV catheter assemblies having a valve and a valve actuator for opening the valve are disclosed.

### BACKGROUND

IV catheters are commonly used for a variety of infusion therapies, including infusing fluids into a patient, withdrawing blood from a patient, or monitoring various parameters of the patient's vascular system. Catheters are typically connected to a catheter adapter that accommodates the attachment of IV tubing to the catheter. Blood control catheters include an internal blood control valve that is opened by the insertion of a male Luer or other object into a proximal end of the catheter adapter. Non-limiting examples of blood control valves are disclosed in United States Patent Application Publication No. 2011/0046570, filed Aug. 20, 2009, titled "Systems and Methods for Providing a Flushable Catheter Assembly." Following placement of the catheter into the vasculature of a patient, an IV fluid source can be connected to the catheter adapter or catheter hub, opening the blood control valve. Thus connected, fluid from the IV source can begin flow into a patient through the catheter.

As is well known in the art, typical blood pressure is 10 to 20 centimeters of water. Infusion bags are usually placed about 100 cm above the patient's heart to direct flow into the patient. At roughly that height, the pressure exerted by the fluid from the infusion bag is much greater than the blood pressure of the patient and therefore can flow into the patient.

Some catheter adapters permit verification of proper placement of the catheter in the blood vessel before fluid infusion begins, such as by providing a flashback chamber of the catheter assembly where a "flashback" of blood can be observed. To confirm flashback in catheter assemblies that do not include a blood control valve, a clinician must manually occlude the vein to prevent undesirable exposure to blood. In contrast, blood control valves can eliminate the need for such manual occlusion, while also reducing the likelihood of blood exposure during catheter placement.

### SUMMARY

Needle assemblies are disclosed, which can include over-the-needle catheter assemblies and safety intravenous catheter (IVC) assemblies. Methods of making needle assemblies and their components form part of the present disclosure.

The needle device can have configurations as described in the various embodiments or can be varied by incorporating features from the described embodiments.

An aspect of the present disclosure can include a catheter assembly, which may more broadly be referred to as a needle assembly, a safety needle assembly, or a needle device.

The catheter assembly can have a blood control system implemented therewith.

The catheter assembly can comprise a catheter hub with a catheter tube attached to a hub body of the catheter hub and a needle hub with a body and a needle having a needle shaft extending through the catheter hub and the catheter tube with a needle tip of the needle extending out a distal end or distal opening of the catheter tube in a ready to use position or ready position.

In the ready position, the catheter assembly can be used to perform a venipuncture or intravenous access.

Before use, a protective cap may be required to be removed from the catheter assembly or needle assembly to expose the needle the catheter tube for venipuncture or intravenous access.

A flashback plug can be provided at the proximal end of the needle hub to allow air to vent but can stop blood from spilling out the proximal opening of the needle hub when blood enters the flashback chamber during primary flashback.

Alternatively, a male medical implement such as a syringe can be attached to the proximal end of the needle hub.

The needle hub can further comprise a shoulder, a tab, or a surface feature that physically contacts the catheter hub, such as the proximal end surface of the catheter hub, to axially register the catheter and needle hubs to set the length of the needle tip projecting out of the distal opening of the catheter tube.

In some examples, rather than the needle hub physically contacting the catheter hub, a third hub can be located between the needle hub and the catheter hub and the needle hub contacting the third hub. When used, the third hub can be a housing. The housing of the third hub can accommodate a needle guard, needle device, biasing element, or spring element for shielding the needle tip from inadvertent needlesticks.

The catheter hub can have a proximal opening and a perimeter defining the proximal opening at a proximal end surface.

The opening at the proximal end of the catheter hub can be sized to receive a male Luer tip. The catheter hub can have external threads and the male Luer tip can have a threaded collar to engage the catheter hub in a Luer lock.

The hub body of the catheter hub can have an interior surface that defines an interior cavity and can have a distal opening at a distal end surface opposite the proximal end surface.

The catheter hub can be a standard catheter hub, a ported catheter hub, or an integrated catheter hub with a tubing extending from a side tubing port.

The catheter hub can include a pair of wings. The pair of wings can incorporate adhesive dressing or medical dressing to facilitate securing the catheter hub to a patient.

A tip protector or needle guard can be provided inside the interior cavity of the catheter hub.

A valve actuator or valve opener can be provided inside the interior cavity of the catheter hub.

In some examples, the valve and the valve opener can be located inside the catheter hub while the needle guard is located outside of the catheter hub, such as being located in a needle guard housing located between the catheter hub and the needle hub.

When the needle assembly or catheter assembly is in a ready to use position, the valve opener or valve actuator is in a first valve opener position. A first valve opener position can be a position in which the valve opener is located in proximity with a valve but does not open the one or more flaps of the valve to enable fluid administration. A second valve opener position can be a position in which the valve opener opens the one or more flaps of the valve to enable fluid administration.

A valve for occluding fluid flow can be provided inside the interior cavity of the catheter hub.

A bushing can be provided inside the interior cavity of the catheter hub.

The needle guard can engage a guard engagement section formed on the interior surface of the catheter hub in the ready to use position and during needle withdrawal prior to activation of the needle guard.

Alternatively, the needle guard can be retained within the catheter hub by one or more distal edges of one or more stabilizer elements or bridges of a valve opener. When retained by the distal edges or bridges, or bridge sections, the guard engagement section, which can be an inward projection, can be omitted from the interior of the catheter hub.

The guard engagement section can have an inside diameter and wherein the inside diameter can be generally constant across a range of diameters of needle shafts from gauge size G18 to G24.

The valve opener can comprise a pair of guide tabs, one each on an exterior of each respective valve opener arm.

The guide tabs can be located in corresponding slots formed in the interior cavity of the catheter hub.

A bridge can connect to two valve opener arms. The bridge can have a distal edge and a proximal edge. The bridge can be formed by two partial bridges. Two partial bridges can have a seam therebetween.

A valve opener can have four partial bridges defining two bridges with each bridge having a seam.

Each of the two valve opener arms of the valve actuator or valve opener can have a first elongated edge, a second elongated edge opposite the first elongated edge, and an end edge. The end edge can be orthogonal to one or both elongated edges.

A first bridge or stabilizer element can connect along two first elongated edges of two valve opener arms.

A second bridge or stabilizer element can connect along two second elongated edges of two valve opener arms.

The first bridge can be formed from two partial bridges or a single continuous bridge.

The second bridge can be formed from two partial bridges or a single continuous bridge.

An actuator head of a valve opener can comprise a frusto-conical shaped distal end.

An aspect of the present invention is a sub-assembly for a needle assembly, said sub-assembly comprising: a valve actuator comprising an actuator head comprising a body having a through opening , two valve opener arms extending proximally of the body, and at least one bridge connected to the two valve opener arms, said at least one bridge comprising a distal edge and a proximal edge; a needle guard comprising a proximal wall having a perimeter defining a proximal opening, two arms extending distally of the proximal wall, and an elbow on each of the two arms; and a needle attached to a needle hub, said needle extending through the needle guard and the valve actuator and comprises a change in profile located proximally of a needle tip; wherein the bridge comprises a seam or is continuously formed without a seam; and wherein the elbow of one of the two arms is positioned distally of the distal edge prior to placement of the needle into a catheter hub.

The sub-assembly can further comprise a guide tab on each of the two valve opener arms.

The bridge of the sub-assembly can include a seam and wherein each of the two valve opener arms can comprise a proximal end and wherein the two proximal ends are spaced from one another a first distance in a first position and spaced from none another a second distance.

The first distance can be greater than the second distance.

The first distance can be smaller than the second distance.

The sub-assembly can further comprise a second bridge connected to the two valve opener arms, wherein the second bridge comprises a seam or is continuously formed without a seam.

The sub-assembly can be assembled to a catheter hub having a catheter tube. A valve can be located inside the catheter hub prior to assembling the sub-assembly to the catheter hub.

The valve actuator used with the sub-assembly can have one or two bridges. The one or two bridges can be continuously formed without a seam or can include a seam.

A valve can be located in an interior of the catheter hub along with the valve opener and the needle guard. The valve can be wedged in a valve seat. The valve can have a valve disc. A skirt section can extend from the valve disc. The valve disc can have one or more slits defining two or more flaps.

A further aspect of the invention is a catheter assembly. The catheter assembly comprises: a catheter hub having hub body with an interior cavity and a catheter tube attached to the hub body; a sub-assembly connected to the catheter hub, said sub-assembly comprising: a valve actuator comprising an actuator head comprising a body having a through opening, two valve opener arms extending proximally of the body, and at least one bridge connected to the two valve opener arms, said at least one bridge comprising a distal edge and a proximal edge; a needle guard comprising a proximal wall having a perimeter defining a proximal opening, two arms extending distally of the proximal wall, and an elbow on each of the two arms; and a needle attached to a needle hub, said needle extending through the needle guard, the valve actuator, and the catheter tube and having a needle tip extending distally of a distal opening of the catheter tube, said needle comprising a change in profile located proximally of the needle tip; wherein the bridge comprises a seam or is continuously formed without a seam; and wherein the elbow of at least one of the two arms is positioned distally of the distal edge.

A valve can be located in the interior cavity of the catheter hub. The valve can be located distally of the needle guard. The valve can be located distally of the valve opener. The valve can have a valve disc with a skirt or without a skirt.

A slot can be formed in the interior cavity of the catheter hub distal of a guard engagement section. The slot can cooperate with a structure formed on the valve opener.

A projection can be formed in the interior cavity of the catheter hub and the seam of the bridge can pass through the projection or the projection can be located at the seam of the bridge.

The valve can comprise a flange defining a circular cavity.

The valve can comprise a plurality of slits and a plurality of flaps. The valve disc can have a thickness. The thickness can vary across the surface of the valve disc.

A still further aspect of the invention is a method of manufacturing a catheter assembly. The method comprises: providing a catheter hub having hub body with an interior cavity and a catheter tube attached to the hub body; connecting a sub-assembly and the catheter hub together, the sub-assembly comprising: a valve actuator comprising an actuator head comprising a body having a through opening, two valve opener arms extending proximally of the body, and at least one bridge connected to the two valve opener arms, said at least one bridge comprising a distal edge and a proximal edge; a needle guard comprising a proximal wall having a perimeter defining a proximal opening, two arms extending distally of the proximal wall, and an elbow on each of the two arms; and a needle attached to a needle hub, said needle extending through the needle guard, the valve actuator, and the catheter tube and having a needle tip extending distally of a distal opening of the catheter tube, said needle comprising a change in profile located proximally of the needle tip ; wherein the bridge comprises a seam or is continuously formed without a seam; and wherein the elbow of at least one of the two arms is positioned distally of the distal edge.

Part of each of the two valve opener arms can extend proximally beyond a proximal edge of a bridge or stabilizer element. For example, the two valve opener arms can each have a proximal end that is positioned proximally of the bridge, and proximally of the proximal edge of the bridge.

A needle guard can be assembled with a valve opener prior to placement of the needle guard and valve opener onto or over a needle.

In other examples, a needle guard can be placed onto or over a needle prior to placement of a valve opener onto or over the needle and around the needle guard.

Placement of a valve opener over or around a needle guard to form a sub-assembly can include moving, tilting, rocking, or displacing the valve opener side-to-side and/or up-and-down while having the needle passing through the actuator head of the valve opener.

Placement of a valve opener over or around a needle guard to form a sub-assembly can include moving, tilting, rocking, or displacing the valve opener side-to-side and/or up-and-down while having the needle passing through the actuator head of the valve opener and a needle passing through a needle guard.

A bridge connecting to two valve opener arms of a valve actuator or opener can have a profile with a generally planar surface or an arcuate surface that extends radially away from a lengthwise axis of the valve opener.

A valve opener can be configured to slide into and push open the valve when advanced by a male Luer tip. The needle shaft of the needle can extend through the needle guard, the valve opener, a valve opening defined by one or more slits of the valve, the bushing, and the catheter tube in a ready to use position.

A valve may be elastically opened so that the needle shaft can pass through the valve opening. The valve can be of the types disclosed in U.S. Patent No. 9,114,231 to Woehr et al.

A catheter hub can include a pair of slots of desired depth formed in the interior cavity of the catheter hub to guide axial displacement of a valve opener when the valve opener is actuated, such as when advanced by a syringe tip or a male Luer tip of an IV administration set or an extension set. In an example, the valve opener can have a projection or projections that move within the pair of slots.

The slots can be spaced from one another, such as equally spaced along the inner circumference of the catheter hub or unequally spaced. The slots can each have a rectangular opening and a length of the opening orientated parallel to the lengthwise axis of the catheter hub.

In other examples, the slots can have other shaped openings. In other examples, there can be only one slot or more than two slots. The number of slots can depend on structures or features incorporated with the valve opener, which can interact with the slots to align or retain the valve opener within the catheter hub.

A groove may be formed inside the interior cavity of the catheter hub to function as a seat for seating a valve, such as to surround and secure an outer perimeter of the valve inside the catheter hub. The groove can be circumferential or ring shape and can be located distally of the slots.

A guard engagement section can be located proximally of the slots and can be provided to secure the needle guard in the ready to use position and during needle withdrawal prior to activation of the needle guard as further discussed below. The guard engagement section can comprise an internal projection, such as a first inside diameter section located adjacent a second inside diameter section, which can be larger than the first inside diameter section.

The internal projection can be continuous or non-continuous, such as made from several spaced sections. The order or location of the groove, the slots, and the guard engagement section can vary from the sequence described to accommodate different valves, valve openers, and/or needle guards. The proximal end of the catheter hub can be sized with a female Luer taper to receive a male Luer tip. The bushing can be configured to retain the catheter tube to the catheter hub. In one example, the bushing can wedge the proximal end of the catheter tube against the interior wall surfaces of the catheter hub to retain the catheter tube to the catheter hub.

The needle guard may embody any number of prior art guards, tip protectors, or safety clips configured for blocking or covering the needle tip of the needle. In the exemplary embodiment shown, the needle guard can embody one of the guards shown in US Pat. No. 6,616,630.

In an example, the needle guard can have a proximal wall having a proximally facing wall surface and a distally facing wall surface with a perimeter defining an opening and two resilient arms. The two resilient arms can extend directly or indirectly from the proximal wall.

The needle can comprise a change in profile, which can be an attachment or sleeve, a crimp, or a bulge, for engaging the perimeter on the proximal wall of the needle guard to retract the needle guard in the proximal direction out of the catheter hub following successful venipuncture, as further discussed below.

In the ready to use position, the proximal wall of the needle guard may be in contact with a distal end of the needle hub, such as to a nose section, a tab, or a fin at a distal end of the needle hub. The two arms of the guard can intersect along a side view as described in US Pat. No. 6,616,630, or they can run along different sides of the needle, or different side edges of the proximal wall, and do not intersect along a side view. In one example, one arm can be longer than the other arm.

Each arm of the needle guard can also include different sections of different arm widths, including a first arm section of a first width and a second arm section of a second width, which is smaller than the first width. The two arms can originate from different ends of the proximal wall and can cross one another at their respective second arm sections. Thus, when viewed from a side along the lengthwise direction of the needle guard, the two arms intersect one another. When used with a needle, the two arms intersect one another when in a ready to use position and when in the protective position.

In an alternative embodiment, the two arms originate from different ends of the proximal wall and extend in a distal direction without crossing one another. Thus, the two arms can also have essentially the same arm width along the length of each respective arm.

The needle guard may be folded from a stamped metal sheet to form the guard as shown herein. Ribs may be formed on the arms, the proximal wall, and/or distal walls to increase structurally rigidity. In some examples, the needle guard can be assembled from different components or parts and can include both metal and non-metal parts.

A distal wall can be provided at an end of each arm of the needle guard. The distal walls on the two arms can overlap one another along an axial direction of the needle guard when the arms close over the needle tip by utilizing different arm lengths and/or angling one of the walls at intersections with the resilient arms so that they are positioned serially along the length of the needle. The intersection between a distal wall and the elongated portion of an arm may also be referred to as an elbow or a diagonal diameter of the needle guard when referring to the two elbows on the two arms.

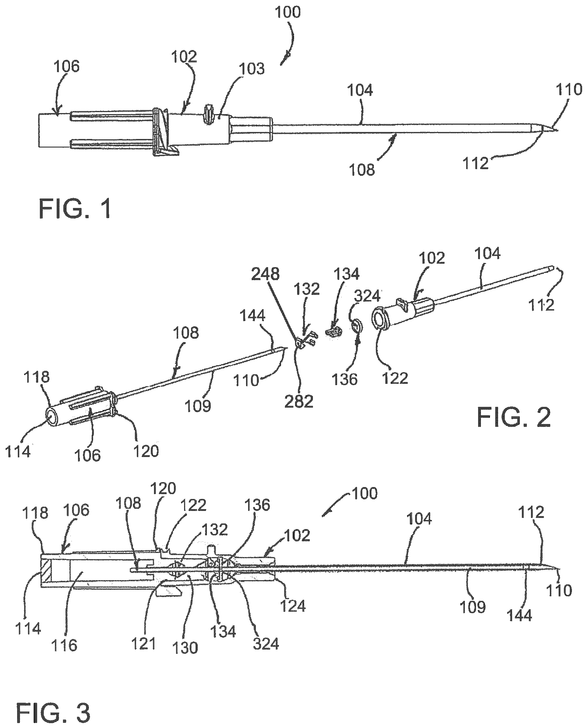

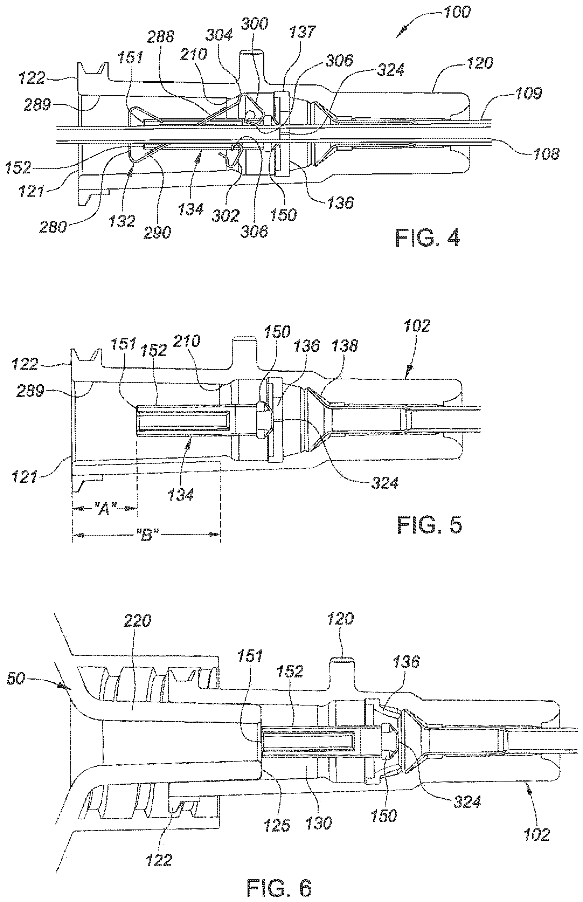

In an example, an intersection or elbow between the distal wall and the resilient arm can engage the guard engagement section inside the catheter hub to secure the needle guard to the catheter hub in the ready to use position, as shown in FIG. 3, and during the process of removing the needle from the catheter hub, as shown in FIG. 4.

The guard engagement section can prevent the needle guard from moving proximally before the needle guard is activated to cover or block the needle tip in the protected position. In some examples, the two distal walls of the needle guard can be called or be considered part of the two arms.

In an example, the needle guard can be positioned inside a valve opener or between two spaced apart structures of the valve opener so that no part of the free ends of the arms, at the ends of the two distal walls, contact or are biased by the valve opener. In other examples, the valve opener can be sized and shaped so as to bias the two free ends of the needle outwardly. When the valve opener biases the arms outwardly, the needle shaft does not have to also bias the two arms outwardly.

The valve opener of the present disclosure can include an actuator head and a pair of valve opener arms. The valve opener arms can be spaced apart and can extend in the proximal direction from the actuator head, which can also be referred to as an actuator head or actuation end for pushing into a valve to open the valve.

The two valve opener arms of a valve opener can be separated from one another by a width, which width can be larger than the diameter of the needle shaft but smaller than the width of the two free ends of the needle guard. Consequently, the two free ends or lips of the needle guard can press against part of the two valve opener arms, which can be called seat clips or arm seats, and be biased outwardly by the two valve opener arms. Thus, when the needle shaft is retracted from the catheter hub following vascular access, there is no drag or friction generated between the two free ends of the needle guard and the needle shaft, such as the exterior of the needle shaft.

During needle withdrawal following successful venipuncture, the change in profile near the needle tip will eventually move proximally against the perimeter defining the opening on the proximal wall of the needle guard. Said differently, following successful venipuncture, the needle and the needle guard can move relative to one another so that the change in profile contacts the proximal wall at the proximal opening of the needle guard. At this moment, additional proximal pulling force by the change in profile on the proximal wall can overcome the engagement between the intersections at the two arms of the needle guard and the guard engagement section of the catheter hub to retract the needle guard proximally out of the catheter hub.

In one example, the additional pulling force of the needle against the proximal wall of the needle guard can cause one or both arms of the needle guard to elastically bend until the intersections can slide through the clearance defined by the guard engagement section of the catheter hub and escape from the engagement. The clearance of the guard engagement section can also be understood as the minimum interior dimension of the catheter hub. That is, the additional pulling force can be applied to cause one or both arms of the needle guard to elastically deflect such that the radial profile of the needle guard at the intersection is reduced to below the minimum diameter of the guard engagement section.

In some examples, the guard engagement section is incorporated with the valve opener and the dimension between two elbows are configured to reduce in size to retract proximally of the guard engagement section of the valve opener. In an example, the guard engagement section of the valve opener is an edge or a bridge, or an edge of a bridge. In other examples, the guard engagement section of the valve opener are two spaced apart edges or two spaced apart bridges, or two edges of two spaced apart bridges.

After the lips of both arms separate from the seat clips or from the guard engagement section of the valve opener, the needle guard can be activated to cover the needle tip. In an example, the two distal walls can move to block the needle tip.

In some examples, the guard engagement section can be omitted and/or the intersections do not engage the guard engagement section in the ready position and during retraction of the needle following vascular access when free ends are biased outwardly by the valve opener.

The guard engagement section can be formed in the interior cavity of the catheter hub to hold the needle guard in the ready to use position and during needle withdrawal, such as when the arms of the needle guard are biased outwardly by the needle shaft. The guard engagement section can extend radially inwardly into the interior cavity of the catheter hub to form a minimum diameter inside the catheter hub.

The guard engagement section can have an interference overlap with the diagonal diameter of the needle guard, such as the two intersections of the two arms, to ensure the needle guard is secured in place inside the catheter hub in the ready to use position and during retraction of the needle. A tapered or slanted surface at the two intersections can decrease the pulling force required to disengage the needle guard from the guard engagement section of the catheter hub or the valve opener, which represent different embodiments of the broad inventive concept.

The needle guard can be assembled inside the interior cavity of the catheter hub by pushing the proximal wall of the needle guard with the distal end of the needle hub as the needle hub with the needle is inserted into the catheter hub. In the ready to use position, the needle guard can extend at least partially into a gap of a valve opener with the proximal section of the needle guard, such as the proximal wall, located outside or proximal of the valve opener.

The proximal wall of the needle guard can be located proximally of the proximal end surfaces of the valve opener. In another example, the proximal wall of the needle guard can be flush or coplanar with the proximal end surfaces of the valve opener. In yet another example, the proximal wall of the needle guard can be located distally of the proximal most surfaces of the valve opener. In embodiments in which the valve opener biases the two arms outwardly, the distal walls of the two arms either do not fit within the gap of the valve opener or the valve opener is provided with seat clips or arm seats to support the two distal walls, as further discussed below.

After the needle hub and needle are withdrawn from the catheter hub and the needle tip is secured by the needle guard, the valve opening of the valve can reseal upon itself to prevent or limit blood or other fluid from passing through the valve opening. That is, after withdrawal of the needle shaft from the valve opening, the potential elastic energy stored in the valve generated from the needle shaft deflecting the flaps of the valve surrounding the valve opening can be released and the flaps return to a less deflected or closed position to reseal the valve opening.

The catheter hub can be designed such that an engagement distance "A" between the proximal end of the catheter hub and the proximal end surfaces of the valve opener may be less than or equal to the minimum length of engagement of a male medical implement, such as a syringe tip or a male Luer fitting, into the catheter hub. This can ensure that in the event of a connection of a male lock fitting, the male lock fitting would introduce a longitudinal force onto the valve opener and allow a longitudinal displacement of the valve opener towards the proximal end of the valve to open the valve. In one example, the engagement distance is per ISO standard, as presently established or established in the future.

The catheter hub can also be designed such that a minimum depth "B" of the interior cavity of the catheter hub from the proximal end of the catheter hub may be greater than or equal to the minimum length of the male lock fitting to ensure that in the event of a connection of a male lock fitting, the male lock fitting would have sufficient surface contact area with the female Luer taper at the proximal opening of the catheter hub.

A male Luer lock or a syringe tip can be inserted into the interior cavity of the catheter hub through the proximal opening at the proximal end of the catheter hub to displace the valve opener in a distal direction to press against the valve to open the valve. In one example, when a male Luer lock fitting is attached from the proximal end of the catheter hub, the male Luer lock fitting can contact the valve opener arms. As the lock fitting is threaded with the exterior threads at the proximal end of the catheter hub, the male fitting pushes against the proximal end surfaces of the valve opener arms distally until the head of the valve opener pushes the valve open.

When the valve opens, fluid can pass through the valve opening of the valve. When the lock fitting is subsequently removed, the valve can elastically recover back to reseal the valve opening. As the valve reseals itself, the valve can stop or limit flow through the opening while simultaneously push the valve opener proximally back to its ready to use position or its proximal position thereby allowing multiple use of the blood control system.

In an example, the actuator head of the valve opener can have a frusto-conical shape so as to readily deflect the flaps of the valve in the distal direction to open the valve. The valve flaps of the valve may be made sufficiently resilient, such as by making the flaps thicker, so as to return to its closed position after the male Luer tip is removed and an axial load is no longer applied to the valve actuator. In another example, an elastic element, such as a resilient gasket or a spring, may be incorporated to assist with closing the flaps on the valve. The frusto-conical shape of the actuator head can retain a proximally directed force vector of the flap against the conical surface, which can push the actuator back to its starting position until the flaps are closed again and flow is stopped. Various embodiments of the valve opener and various embodiments of the valve, which may be implemented in the blood control system, are further discussed below.

In another example, a valve opener can comprise an actuator head and at least one valve opener arm, such as a leg element or an elongated extension. In an exemplary embodiment, two valve opener arms can extend from the head in the proximal direction and each can have a length measured in a lengthwise direction of the catheter assembly and a width, measured orthogonally to the length. As shown, the two valve opener arms can be spaced apart and extending from opposite points or sections of the head. The two valve opener arms can be sized and shaped for contact by a male Luer to transfer a distally directed force from the male Luer to the head to then push against the valve to open the valve.

A bridge or stabilizer element connected to the two valve opener arms can add rigidity to the two valve opener arms and provide additional surface area for contact with the male Luer. The bridge can extend from an outer edge of one valve opener arm to an adjacent outer edge of the other valve opener arm to connect the two valve opener arms together without impeding fluid flow through the valve opener or interfering with the needle or the needle guard, which can be located between the two valve opener arms in the ready to use position.

The outer surfaces of the two valve opener arms and the bridge can be arc shaped and piecewise continuous to form a semi or partial circular shaped cross section along a width or radial direction of the valve opener. In an example, proximal surfaces of the valve opener arms and a proximal surface of the bridge can be coplanar. In other examples, the proximal surface of the bridge can locate distally of the proximal surfaces of the two valve opener arms.

In some examples, the outer surfaces of the two valve opener arms can also be tapered in the lengthwise direction to match a taper of the interior cavity of the catheter hub. In one example, the two valve opener arms can taper inwardly from the proximal surfaces of the valve opener arms to match the female Luer taper at the proximal end of the catheter hub. The bridge can extend a short distance in the distal direction to provide a through opening or through hole cooperatively defined between the bridge, the two valve opener arms, and the head.

The through opening can be provided to enable an intersection of one of the arms of the needle guard to project therethrough to engage the guard engagement section of the catheter hub. Alternatively or additionally, the intersection can engage the distal edge of the bridge to retain the guard in the ready to use position and during retraction of the needle. In some examples, the bridge can extend to the head and no through opening or through hole is provided between the two valve opener arms and the bridge. The thickness of the bridge can be sufficiently thin such that during assembly, at least one of the arms of the needle guard can elastically deform to move pass the bridge along the longitudinal axis without causing permanent deformation or damage to the needle guard during assembly and during activation of the needle guard.

The thickness of each of two valve opener arms can be sufficiently small or thin so that the needle guard and the two valve opener arms have sufficient clearance to fit within the interior cross-sectional space of the catheter hub without being physically binding against the catheter hub and rendered unmovable or fixed. In an example, the thickness of each of two valve opener arms and the width of the needle guard are such that no undercut or channel is required to be formed in the interior wall surfaces of the catheter hub to accommodate them. When the valve opener arm has an arc cross section, it may be structurally stronger to handle a greater load when being pushed by a male tip to push the head against the valve. This can allow a thin and compact design for the infusion device and gives more room in the standardized space of a female Luer taper.

The valve opener can be made from a metal material or from a polymer material. When made from a metal material, the valve opener can be formed by deep draw methods or punching or cutting a pattern from a sheet metal and bending the stamped or cut pattern to form features of the valve opener including the head and the at least one valve opener arm. Some welding to join individual components are contemplated. The at least one valve opener arm can be bent further to form an arc-shaped cross section to provide added rigidity when pushed by the male Luer. In one example, the valve opener is formed from a stamped stainless steel pattern worked into a desired shape. When made from a polymer material, the valve opener can be molded as a single piece. In some examples, the valve opener can be co-molded or insert molded to form surface features and/or to incorporate different materials. In one example, the polymer material can be a high-strength semi-rigid or rigid material.

An undercut can be formed on the exterior circumference of the valve opener and can be located on the exterior of the valve opener arms. The undercut can be sized, shaped, and located on the valve opener so that it seats distal of the guard engagement section or so that the guard engagement section can secure the valve opener within the interior of the catheter hub from displacing proximally out of the catheter hub. The configuration of the undercut, such as the curve, the dimension, the width, the height, etc., can be selected to allow easy axial displacement of the valve opener towards the valve and back and be retained by the guard engagement section.

The undercut on the valve opener can have two sidewalls separated by a bottom surface, similar to a recess or groove. The two sidewalls can be perpendicular to the bottom surface or tapered outwardly to form an opening wider than the width of the bottom surface. The depth of the undercut can allow the valve opener to slide freely. The amount of axial displacement can be limited by the width of the bottom surface or the sidewalls interacting with the guard engagement section. The shape of the undercut and its relative position to the guard engagement section can also cause the actuator head to contact and/or provide a slight axial load against the proximally facing surface of the valve even when the valve opener is not axially loaded or pushed by a male Luer tip.

The actuator head of the valve opener can comprise a body with an outer perimeter. In an example, the outer perimeter can be generally rectangular with two opposing flat surfaces that align with the opening of the gap. The perimeter further comprises two opposing arc-shaped side surfaces, which are continuous with the arc-shaped surfaces of the valve opener arm. In other examples, the outer perimeter can have a taper extending outwardly from a distal end to a proximal end to form a wedge. Interiorly, the body can comprise a through opening formed through the head and in fluid communication with the gap to allow fluid to pass through the valve opener.

In an alternative embodiment, the distal side or surface of the actuator head of the valve opener can include a taper or frusto-conical nose section to facilitate opening the valve, such as to deflect the flaps of the valve.

In another example, a valve opener can comprise a head and a pair of valve opener arms, which may also be referred to as leg elements or elongated extensions, extending in a proximal direction, either directly or indirectly, from the head. The valve opener can have a head or a nose section that is shaped differently to generate a different deflection on the valve to open the valve and to aid in retracting the valve opener proximally when the male medical implement is removed, which allows the valve to close.

The valve opener can be without any bridge. Additionally, a pair of guide tabs can extend from opposite sides of the valve opener, such as radially of the two valve opener arms, to restrict movement of the valve opener to an axial direction within a limited range. For example, the guide tabs can cooperate with the slots in the interior cavity of the catheter hub to confine movement of the guide tabs therein and hence movement of the valve opener.

Upon insertion of the valve opener into the catheter hub during installation of the valve opener, the guide tabs can create a sound, such as a click, to provide audible feedback. The shape of the guide tabs and their relative positions to the slots can also cause the actuator head to contact and/or provide a slight axial load against the proximally facing surface of the valve even when the valve opener is not axially loaded or pushed by a male Luer tip.

The two valve opener arms can attach at their respective distal ends to base members of the actuator head. Each base member can have a tapered surface along the interior and a tapered surface on the exterior, relative to the gap located in the interior. As shown, the guide tabs extend from the exterior tapered surfaces. In a particular example, the guide tabs can be located at the proximal ends of the two exterior tapered surfaces.

The frusto-conical shaped head can have a flat circular front surface transverse to the needle axis at a distal end of the head and a conical tapered surface extending proximally and outwardly from the flat circular front surface to form a conical wedge. The conical shape of the head can assist the valve opener to push open the valve and retract proximally upon the valve closing.

A guide structure can be formed on an exterior of each valve opener arm just proximal of the conical tapered surface. The guide structure can form by extending the conical tapered surface of the head and a sharp transition or indentation to define a shoulder on the exterior of each arm. Said differently, the proximal end of the conical tapered of the head can have an outside diameter of a first dimension and the two valve opener arms can define an outside diameter of a second dimension, which is smaller than the first dimension, to define the two guide structures. The first dimension can be larger than the circumference of the projection in the interior cavity of the catheter hub. The guide structure can be provided between the two different dimensions on each arm. Alternatively, the guide structures can extend outwardly anywhere along a length of the valve opener from opposite sides of the valve opener.

The first dimension at the proximal end of the conical tapered surface of the head can be around 10% to 40% smaller than an outer diameter of the valve to ensure that the head of the valve opener would not pass fully through the slits of the opened valve when a male Luer fitting advances the valve opener. This can allow the valve opener to return or retract proximally upon removal of the male Luer and the valve closing, thus allowing multiple use of the blood control system. Alternatively, the valve opener can be sized and shaped so that the guide structures can pass through the opened valve and be stuck distally of the valve making the valve and valve opener a one-time use blood control device. The guide structures and the head can cooperatively form an arrow like structure with a truncated tip.

Horizontal slots can be provided inside the interior cavity of the catheter hub distal of the guard engagement section to guide the valve opener in the axial direction inside the interior cavity. That is, the guide structures can be configured to slide axially back and forth inside horizontal slots formed in the interior cavity of the catheter hub. The shape of the guide structures and their relative positions to the slots can also cause the actuator head to contact and/or provide a slight axial load against the proximally facing surface of the valve even when the valve opener is not axially loaded or pushed by a male Luer tip.

Fingers can extend distally from a center portion of the head that together can define a distal projection having a through passage therebetween. The fingers can form by slitting the surface of the head and then bending the tabs to create fingers that define the distal projection. In an example, there can be four fingers that can form a generally square or rectangular projection. The fingers can act as an opening member to push open the valve when the valve opener is pressed distally into the valve by a male Luer fitting. The shape and number of fingers can vary.

Side wings can extend outwardly and transversely from outside surfaces of the valve opener arms. The side wings can extend transversely from the valve opener arms to form an angle greater between zero degrees and 90 degrees to allow the valve opener to snap-fit into the horizontal slots of the catheter hub. Thus, the side wings are similar to the guide structures on other valve openers described elsewhere and can extend outwardly anywhere along a length of the valve opener of the present embodiment from opposite sides of the valve opener.

A proximal flange can extend outwardly along the transverse axis from the proximal end surface of each valve opener arm to provide additional surface areas for the male Luer conical fitting to press against to push the valve opener in the distal direction to open the valve. Arm flanges can extend outwardly and transversely on opposite lengthwise edges of the valve opener arms to increase the structural rigidity of the valve opener arms. In some examples, the surface of the opener arms can be worked to for a curved shape, such as an arc shape, to increase the strength of the arms against axial load by the male Luer tip.

A valve may be usable with the catheter assemblies and hubs with a female Luer described elsewhere herein. The valve can be called or considered a disc valve. The disc valve can optionally include one or more flanges extending therefrom, as further discussed below. The valve can have a valve body having a width measured from one edge to another edge of an outer perimeter or outer diameter of the valve body. The valve body can have a thickness, which is the dimension that extends orthogonal to the width from a first surface to a second surface. The valve can be seated in a valve seat area or valve groove provided in the interior cavity of the catheter hub. The valve can be configured to be located distal a valve opener to form a seal in the interior cavity and to be opened by the valve opener.

The valve groove can be a radial undercut formed in the interior cavity of the catheter hub with a depth in the range between about 0mm to about 1mm deep, or recessed, to allow proper seating. The depth range can have a tighter tolerance of about 0mm to about 0.3mm. A seal can be provided between the interior of the catheter hub and the outer surfaces of the valve such as the outer perimeter.

In one example, a slight compressive force can be applied by the catheter hub against the perimeter of the valve to ensure sealing. Alternatively or in addition, the outer edges of the first and second surfaces of the valve body can seal against the surfaces of the valve groove to provide a seal with the catheter hub. In an example, the profile of the valve groove as well as any profile internally of the catheter hub, such as slots or projection(s), can be contoured with radiuses to minimize smearing of material during the molding process of the catheter hub.

A valve opening can extend through the thickness of the valve body from the first surface to the opposite second surface. The thickness of the valve body can be uniform or can vary. The thinner the valve, the less resistance the valve is to opening, and therefore less force required to open the valve. The thicker the valve, the more elastic energy is able to be stored to enable the valve to return to its original pre-opened shape upon elastic deformation, and thus allowing multiple access of the valve opening and closing. In one example, the thickness of the valve is in the range of about 0.3mm to about 1.5mm. However, the thickness can vary and can include other ranges.

A first flange can extend axially from the outer perimeter or outer diameter of the valve body to allow better seating of the valve in the catheter hub. The first flange can be oriented in the catheter hub to extend proximally or distally. The first flange and the valve body can cooperatively define a first circular cavity concentric with the valve body and locally reducing the thickness of the valve at the first circular cavity.

The valve opening can include or comprise three slits extending radially from a center of the valve and formed approximately degrees apart, thereby forming a first flap, a second flap, and a third flap. That is, the three slits can intersect at a single central point coinciding with the axis of the valve. In other examples, the slits can be unequally spaced apart. The length of the slits can vary. In one example, the slits extend to the first flange. The first flap, the second flap, and the third flap can be deflected to open a flow path through the valve body. The fluid flow path can be provided when the three flaps are deflected by the valve opener. In an example, the flaps near the central point can expand radially towards the perimeter and in the distal direction when deflected by the valve opener. That is, the first flap, the second flap, and the third flap can be deflected by pushing the valve with one of the valve openers described herein on a proximal side of the valve.

Alternatively, the valve opening can be a single slit formed through the thickness of the valve body and defining a first flap and a second flap, which can also be deflected to open a flow path through the valve body by pushing the valve with a valve opener on one side of the valve.

In an example, the valve opening may also include reliefs embodying two short through cuts at each end of the slit forming a V-shaped relief. The reliefs can provide clearance for the flaps to enable them to deflect more readily when pushed open by the valve opener. Less preferably, a single short through cut may be incorporated at each end of the slit.

A second flange can extend axially from the outer perimeter of the valve opposite the first flange thereby cooperatively forming a second circular cavity concentric with the valve body and further reducing the thickness of the valve at the first and second circular cavities.

An alternative valve similar to other valves described elsewhere herein can comprise a circular valve body with a varying thickness that increases from a central point of the valve towards the outer perimeter of the valve. The thickness can vary linearly with a constant slope or can have a complex slope. The valve can have an opening with three slits provided through the thickness of the valve body to form three flaps. The three slits can intersect at a single central point coinciding with the axis of the valve. The flaps can be deflected to open a flow path through the valve body. The first, second and third flaps can be deflected by pushing the valve with a valve opener to deflect the flaps radially and axially. A fluid flow path can be provided when the three flaps are deflected. In an example, the flaps near the central point can expand radially towards the outer perimeter and in the distal direction when deflected by the valve opener pressing against the proximally facing surface of the valve.

The valve can be located inside the catheter hub just distal of the head of the valve opener. The valve can comprise an outer perimeter that can be seated in a valve groove to fix the outer perimeter of the valve between the valve opener and the bushing. Alternatively, the outer perimeter of the valve can be fixed to the interior cavity of the catheter hub between the valve opener and the bushing by interference fit, adhesive, or other securing means.

To remove the needle from the catheter hub with the needle guard covering the needle tip in a protective position, the needle device starts at the position of FIG. 3, which may first require removal of a disposable protective cap, moves to a transition position of FIG. 4 wherein the needle slides proximally relative to the catheter tube and the catheter hub, and then continuing to move the needle until the needle tip moves proximally of two distal walls, one on each end of the resilient arms of the needle guard. The distal walls can be considered part of the arms on the needle guard and are specifically called out so as to identify the structure and function in relations to the other components and how the disclosed needle assemblies operate.

Where the needle guard has only one distal wall and/or one arm, the process is similar but the needle tip only has to move proximally of the one distal wall to cause the needle guard to activate. As the two distal walls and hence the two resilient arms are no longer biased outwardly by the needle or valve opener, the two arms move radially to decrease the guard's radial profile and to disengage from the guard engagement section of the catheter hub. Alternatively, the one arm and one distal wall can disengage from the one guard engagement section.

As the needle continues to move in the proximal direction and the change in profile engages the perimeter on the proximal wall of the needle guard, the needle guard is moved proximally with the needle. Alternatively the needle guard can clamp onto the needle shaft and be removed from the catheter hub as a unit without utilizing a needle crimp. Note that in the protective position in which the needle guard covers the needle tip, the valve and the valve opener remain inside the interior cavity of the catheter hub. Thus, the valve and the valve opener can locate inside the catheter hub in both the ready position of the needle and the protective position of the needle. Viewed from another perspective, the valve and the valve opener can be located inside the catheter hub in both the ready to use position of the catheter assembly, in which the needle tip projects out a distal opening of the catheter tube, and a protective position of the catheter assembly, in which the needle is removed from the catheter hub and the needle tip is covered by a needle guard.

A male medical implement or instrument can be a male Luer, a syringe tip, an IV set connector, or other male tip having a Luer taper. The male medical implement can be located in the proximal opening of a catheter hub. For example, the male medical implement can be connected to an IV tubing, which is connected to an IV fluid source for fluid delivery through the male medical implement, the catheter hub, and the catheter tubing to deliver fluid therapy to a patient.

When initially inserting the male medical implement, such as a male tip, into the proximal opening of the catheter hub, the male tip can initially contact the two valve opener arms on the valve opener to advance a distally directed force on the two valve opener arms to open the valve. The proximal end surfaces of the valve opener arms can provide a contact surface for the distal end of the male medical instrument, as previously discussed.

The valve opener arms can also be designed to contact the inside wall of the catheter hub at a tangential point. In this way, the valve opener arms can be stable and can resist being deflected outwards. This arrangement can avoid relatively thin valve opener arms from wedging between the male medical instrument and the inside wall of the catheter hub. The distally directed force moves the valve opener in the distal direction until the geometries of the male tip and the proximal opening of the catheter hub stop further distal advancement of the male tip. In an example, a female Luer taper of the catheter hub and a male Luer taper of the male tip can register to form a Luer engagement and block distal advancement of the male tip further into the opening of the catheter hub. A seal can be provided by the Luer engagement to prevent fluid from leaking out the proximal opening of the catheter hub.

As the valve opener moves distally by the distal advancement of the male tip, the head of the valve opener is urged distally and pushes against the proximally facing surface of the valve. In particular, the distal end of the valve opener initially pushes against the proximally facing surface of the valve. As the valve is fixed inside the catheter hub, the flaps of the valve are urged distally by the valve opener, which is urged distally by the male tip. For example, the head can contact and push the valve in the distal direction thereby opening a flow path through the valve opening of the valve. Fluid from the male tip can then flow through the catheter hub, through the valve, and through the lumen of the catheter tube. Alternatively, a suction can be applied by the male medical instrument, such as a syringe or vacuum blood collection tube, and blood aspirated from the patient. This is often done for testing samples before infusion therapy is commenced. Also, typically any remaining blood is first flushed from the inside of the catheter hub before infusion therapy is commenced.

The shape of the head of the valve opener or features on the head of the various valve openers can facilitate deflection of the flaps on the valve radially outwardly and in the distal direction, and can facilitate retracting the valve opener in the proximal direction to close or seal the valve. The heads can be designed as a one-time use. That is, the heads of the various valve openers can be designed such that they stick to the valve or contact the valve in such a way that does not allow the flaps to uncoil even after removal of the male Luer tip and the no axial load is applied on the valve opener.

To change the male tip or to simply close the valve from the open position, the male tip can be removed in the proximal direction away from the catheter hub, which removes the axial load on the valve opener. The biasing or resilient nature of the valve, which can be made from an elastomer, allows the valve to recoil to its more relaxed state. Thus, the flaps on the valve will recoil by moving proximally, which pushes the valve opener in the proximal direction inside the interior cavity of the catheter hub. The valve opener therefore can return to its original position after removal of the male tip from the catheter hub. In some examples, an elastic gasket or a helical spring may be used in the interior distal chamber of the catheter hub, distal of the valve, to help push the flaps close upon removal of the male Luer tip.

Methods of making the catheter assemblies and their components described elsewhere herein are within the scope of the present disclosure.

The needle guard can engage a guard engagement section formed on the interior surface of the catheter hub in a ready to use position and during needle withdrawal prior to activation of the needle guard.

The guard engagement section can have an inside diameter and wherein the inside diameter can be generally constant whether used with any number having a needle diameter from G18 to G24.

The valve opener can comprise a pair of guide tabs, one each on an exterior of each respective valve opener arm.

The guide tabs can be located in corresponding slots formed in the interior cavity of the catheter hub.

The guide tabs can produce a sound when engaging the slots. The sound can be a click. The click can emanate from one guide tab or from both guide tabs.

A bridge can connect two valve opener arms together. The bridge can have an arc shape and the arc shape can contact the interior surface of the catheter hub or be spaced from the interior surface. The bridge can add strength to the two valve opener arms to prevent excessive deflection or buckling. In some examples, there can be two bridges incorporated with the valve opener.

In some examples, a bridge can be formed by two partial bridges and has a seam or a gap between the two partial bridges. In some example, a bridge can be formed as a continuous structure without a seam.

The head of the valve opener can comprise a frusto-conical shaped distal end.

The method can be used a needle shaft having a diameter of a G18 needle.

Using a catheter hub with the same inside minimum diameter and the same valve opener, the method can be used a needle having a diameter of a G20, G22, or G24 needle.

The method wherein the needle assembly is a first needle assembly and the method can comprise making a second needle assembly identical to the first needle assembly and wherein the needle shaft of the second needle assembly has a diameter of a G20 needle, G22 needle, or G24 needle.

Aspects of the present disclosure can include a needle assembly comprising a needle hub with a needle extending from a distal end of the needle hub, a catheter hub having an interior cavity, a catheter tube attached to the catheter hub and having the needle extending through the catheter tube in a ready to use position, a valve seated in the interior cavity of the catheter hub, a needle guard comprising at least one arm extending from a proximal wall, and a valve opener positioned in the interior cavity of the catheter hub and proximal of the valve in a first position, the valve opener comprising a head distal the needle guard, and one or more valve opener arms extending in a proximal direction of the head between the needle guard and the interior cavity of the catheter hub, and wherein the head of the valve opener can be movable through an opening of the valve in a second position.

The needle guard can engage a guard engagement section in a ready to use position and during needle withdrawal prior to activation of the needle guard.

The guard engagement section can extend from the interior cavity of the catheter hub.

The at least one arm can engage the needle shaft in the ready to use position and during needle withdrawal prior to activation of the needle guard.

The at least one arm can engage the one or more valve opener arms of the valve opener in the ready to use position and during needle withdrawal prior to activation of the needle guard.

The guard engagement section can extend from the one or more valve opener arms.

The one or more valve opener arms can be a pair of valve opener arms at opposite sides of the valve opener.

The one or more valve opener arms can be a pair of valve opener arms at opposite sides of the valve opener.

The valve opener can further comprise a shoulder formed between the head and each valve opener arm.

The shoulders can be received in horizontal slots defined in opposite sides of the interior cavity of the catheter hub. The horizontal slots can limit the range of axial movement of the valve opener.

The valve opener can be biased proximally from the second position to the first position by the valve.

Another aspect of the present disclosure can include a catheter assembly comprising a catheter hub having a proximal opening extending distally into an interior cavity, a catheter tube attached to a distal end of the catheter hub, a valve seated in the interior cavity of the catheter hub, a valve opener positioned proximal of the valve in the interior cavity of the catheter hub, the valve opener comprising a head and a pair of valve opener arms extending proximally of the head, a needle guard extending into a gap between the valve opener arms and comprising at least one arm extending from a proximal wall, the needle guard, and wherein the valve opener is extendable through an opening of the valve.

A guard engagement section can extend from the interior cavity of the catheter hub to secure the needle guard in a ready to use position.

The guard engagement section can form a minimum diameter of the interior cavity of the catheter hub.

The needle guard can bias against the needle shaft in the ready to use position and during needle withdrawal.

A guard engagement section can extend from the pair of valve opener arms to secure the needle guard in a ready to use position.

The needle guard can bias against the pair of valve opener arms in a ready to use position.

A shoulder can be formed between the head and each of the pair of valve opener arms.

The shoulders can be received in horizontal slots defined in opposite sides of the interior cavity of the catheter hub. The horizontal slots can limit the range of axial movement of the valve opener.

The head of the valve opener can be tapered.

A valve opener can comprise an actuator head, sometimes referred to its abbreviated form as head, comprising a through opening and a pair of valve opener arms, which may also be referred to as leg elements or elongated extensions, extending in a proximal direction, either directly or indirectly, from the head. The through opening at the actuator head can be circumscribed by a solid structure forming completely around the opening or the opening can be partially surrounded, such as by including a slit or channel through the body of the actuator head.

The through opening allows the needle to pass therethrough during assembly in a ready to use position and for fluid to flow through during fluid administration following successful venipuncture or intravenous access.

The actuator head can have a generally cylindrical shaped body with an optional radiused distal end and the two valve opener arms. The body of the actuator head can have a generally cylindrical shape or can have segments or edges, like a polygon when viewing an end section.

Each opener arm can have a proximal end and the two proximal ends of the two arms can be spaced from one another a first distance in a first position of the two opener arms and can be pressed or moved together closer to one another a second distance in a second position of the two opener arms, and wherein the second distance can be smaller than the first distance. In some examples, the two valve opener arms can be moved further away and the second distance can be greater than the first distance.

The valve opener of the present embodiment can be fabricated with valve opener arms that are spread apart at their respective proximal ends to facilitate coupling or mounting with a needle guard and that then move together to close over the needle guard. This configuration of the valve opener can minimize compression or distortion of the needle guard when assembly the needle assembly or catheter assembly. Among other things, the spaced apart valve opener arms can function as an enlarged entrance or opening for coupling the needle guard and the valve opener together during assembly.

The actuator head can be shaped for one-time actuation when advanced by a male Luer tip following successful venipuncture to open the valve. For example, the body of the actuator head can project through the one or more slits of a valve and remain attached to the valve even after the male Luer tip is removed.

In some examples, the valve can be configured with sufficient elastic force and/or the body of the actuator head can be sized with a greater taper for the valve flaps to impart component forces to move the valve actuator in a proximal direction after removal of the male Luer tip, for a multi-use valve system.

Optionally or additionally, a helical extension spring or an elastic gasket may be used with a valve and valve opener or actuator combination to provide an external axial force to assist the valve actuator to move away from the valve and in the proximal direction after removal of the male Luer tip from the proximal end of the catheter hub.

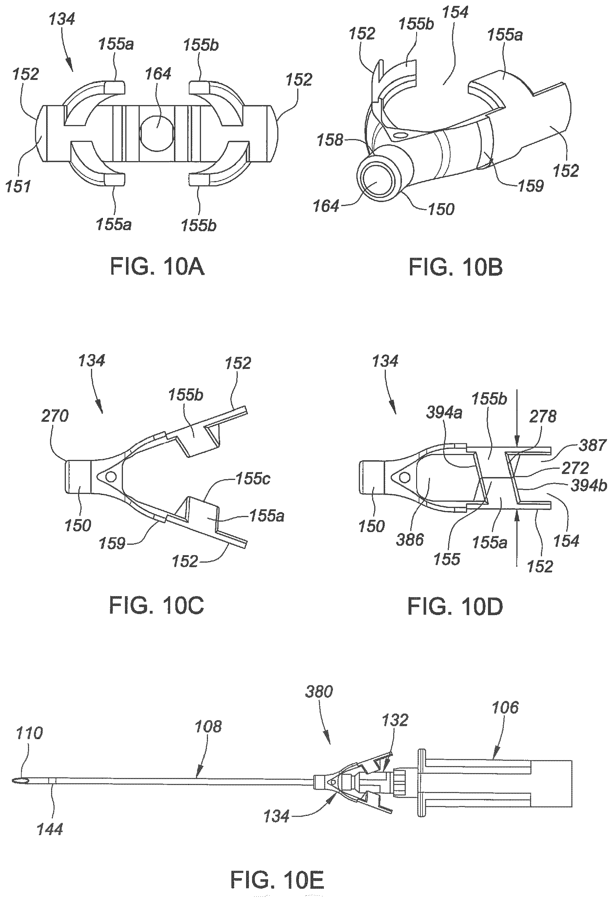

The valve opener can be fabricated with the two valve opener arms radially spaced from one another, when viewing the arms' respective proximal ends. In this spaced configuration, as shown in FIGs. 10A-10C, an enlarged gap can be defined by or can be provided between the two spaced apart actuator arms or valve opener arms in the arms' first position. However, when the two arms are moved together in the arms' second position, as shown in FIG. 10D, the gap between the two arms can decrease.

A valve actuator can have a gap between two valve opener arms having a first dimension, in which the two arms are in their respective first positions, and a gap between the two valve opener arms having a second dimension, in which the two arms are in the respective second positions. The gap in the second position with the second dimension can be smaller than the first dimension.

The larger first dimension of the gap, due to the two valve opener arms being spaced from one another at their respective proximal ends, can provide ample space for coupling the valve opener with a needle guard, as further discussed below. Thus, the valve opener can be configured to facilitate assembly by providing an enlarged gap, or opening, in a first configuration of the valve opener and that can then be reduced in a second configuration of the valve opener after pairing the valve opener with the needle guard.

The arrangement with movable valve opener arms allows for the assembly of the valve actuator and needle guard to be performed while minimizing bending and/or compressing the needle guard to get the arms of the needle guard, and the distal walls of the needle guard, through the opening defined by the two actuator arms and the two bridges of the valve actuator, which can damage the needle guard.

Two bridges or stabilizer elements can be incorporated connecting to the two valve opener arms. The valve opener can have a distal cavity opening or chamber 386 and a proximal cavity opening or chamber. The two bridges can be formed from partial bridges or partial stabilizer elements, or alternatively from continuously formed structures forming the bridges.

In an example, each of the two actuator arms can comprise a pair of partial bridges or stabilizer elements. When the two valve opener arms are moved closer together in their respective second positions, the four partial bridges or partial stabilizer elements can form two bridges or stabilizer elements each with a seam. In an example, two adjacent partial bridges can be secured to one another after they contact to form a bridge without an openable seam, such as by bonding, gluing, or welding.

In still other examples, the partial bridges can be provided with detents along the inner edges for snapping together in mechanical engagement.

In another example, the adjacent partial bridges are not secured to one another after they contact to form a bridge without an openable seam but are constrained by the interior surface of the catheter hub when the valve actuator is placed inside the catheter hub.

When the valve opener arms are moved together to form one or two bridges, with or without openable seams, an opening is defined by the two bridges and the two actuator arms. To move an object from the proximal cavity opening or chamber to the distal cavity opening or chamber between the two bridges in the configuration shown in FIG. 10D, the object needs to travel through the opening defined by the two bridges and the two valve opener arms. Consequently, an object having a profile that is larger than the largest dimension of the opening must compress or be distorted to be smaller than the opening in order to pass therethrough.

Each bridge or stabilizer element can comprise a distal edge and a proximal edge. In an example, the distal and proximal edges of each respective bridge are parallel to one another. Relative to the lengthwise axis of the valve actuator, the distal and proximal edges can be orthogonal or be angled to the lengthwise axis. Further and comparing to the edges of the opposing or other bridge, the edges can have different angles from one another or be parallel to one another.

The distal edges of the two stabilizer elements can provide structural surfaces for the intersection or elbow between the distal wall and the resilient arm of each of the two arms to engage or abut to prevent the needle guard from moving in the proximal direction in a ready to use position and during retraction of the needle following successful venipuncture.

Two stabilizer elements or bridges can retain the needle guard to the valve actuator in both the ready position and during retraction of the needle following successful venipuncture. By engaging the needle guard to the two bridges or by providing a barrier to the needle guard, and more particularly by utilizing the two distal edges of the two bridges, or by arranging the two bridges to block proximal movement of the needle guard, the needle guard does not need to engage the guard engagement section formed inside the catheter hub as described elsewhere.

However, when two arms of a needle guard are no longer biased outwardly by the needle, or kept apart by the needle, the diagonal dimension at the two elbows of the needle guard decreases to a dimension that is smaller than the opening size at the two bridges to then allow the needle guard to move proximally of the two bridges, and not be impeded by the distal edges of the two bridges.

A valve actuator may be made from plastic injection molding with two actuator arms and partial bridges of the valve actuator formed in their first spaced apart positions, where partial bridges from two valve opener arms do not contact. In moving the valve opener arms together if they are formed in an original spaced apart position, the arms can experience strain and can spring back open to their relaxed state. In an example, the two arms can be secured after moving together by bonding or gluing the two sets of partial bridges.

Alternatively, the two arms can be held closer together by placing the valve actuator inside a catheter hub and the interior surface of the catheter hub constraining the two arms. Depending on the clearance between the interior of the catheter hub and the valve actuator, the two arms of the valve actuator may naturally move away from one another inside the catheter hub. Consequently, when constrained only by the catheter hub, the seams at the two bridges can widen to more than a line.

Exteriorly, guide tabs can be provided on each of the two actuator arms. The guide tabs can cooperate with surface features inside the catheter hub for alignment purposes and to retain the valve actuator inside the catheter hub. For example, the two tabs can cooperate with a pair of slots in the interior of a catheter hub to prevent the valve actuator from rotating about the lengthwise axis of the catheter hub. The pair of slots can also retain the valve actuator from displacing proximally out the proximal opening of the catheter hub.

Optionally or alternatively, different surface structures, different from the pair of slots, inside the catheter hub can be implemented to retain the valve actuator from displacing proximally out the proximal opening of the catheter hub. For example, a different set of projections, lips, or shoulders can be incorporated inside the catheter hub to limit proximal movement of the valve actuator.

A valve opener and a needle guard can be mounted on a needle and the needle attached to a needle hub. When the needle guard is mounted onto a needle, the needle biases the two arms of the needle guard outwardly. Where the needle guard incorporates a single arm, the single arm is biased by the needle outwardly.

A sub-assembly can be formed by first securing the needle to the needle hub and then placing the needle guard onto the needle before adding the change in profile near the needle tip of the needle, such as by crimping to form a crimp. Alternatively, the needle guard can first be placed on the needle and then the needle attached to the needle hub prior to forming the change in profile.

A needle guard can be placed onto a needle by directing the proximal end of the needle, opposite the end with the needle tip, through the distal end of the needle guard and continuing until the proximal end of the needle projects through the opening at the proximal wall of the needle guard.

Next, the valve opener can slide onto to the needle having the needle guard with the two spaced apart actuator arms of the valve opener moved directly over the needle guard and ensuring that the elbows of the two arms are distal of the partial bridges when the arms are moved closer together to their respective second positions.

The through opening at the body of the actuator head is sufficiently wide or large to readily slide onto the needle and over the change in profile without any interference. The partially assembled components may be referred to as a needle, guard, and actuator sub-assembly, or an NGA sub-assembly, which can also be referred to its abbreviated name as simply a sub-assembly. The sub-assembly is so formed prior to assembling with a catheter hub having a catheter tube, and prior to placing the valve opener into the interior of the catheter hub.

The NGA sub-assembly can be partially inserted into a catheter hub having a catheter tube attached thereto and continuing to form a catheter assembly. During insertion of the NGA sub-assembly, the two actuator arms can be held together in their respective second positions to provide clearance for insertion of the valve actuator into the proximal opening of the catheter hub. As previously described, the two actuator arms can be held together by gluing or bonding the partial stabilizer elements. In other examples, detents may be use to engage the partial stabilizer elements to keep the arms closer together during assembly.

A catheter assembly having a valve actuator and a needle guard secured inside a catheter hub without having to compress or deform the two arms of the needle guard to assemble the needle guard to the catheter assembly can be implemented or formed utilizing valve openers of the present disclosure. In an example, a valve actuator can be located proximally of a valve. The valve can have a skirt defining a cavity and the actuator head of the valve opener can be located in the cavity of the skirt.

In an alternative embodiment, the valve can be without a skirt. The nose section of the needle hub can be located at the proximal end in the proximal opening of the catheter hub. In an example, the nose section of the needle hub can be used to push the proximal ends of the valve opener into position inside the catheter hub.

In an alternative embodiment, two actuator arms can be fabricated in a normal extended position from the actuator head, and not outwardly spaced apart at their respective proximal ends to then be moved closer together. Further, partial stabilizer elements incorporated with the valve actuator can be shorter along the radial direction compared to partial stabilizer elements disclosed in other embodiments. Thus, the actuator arms are not intended to deflect radially outwardly or radially inwardly relative to a lengthwise axis of the valve actuator although the arms can deflect since the partial stabilizer elements are not configured to abut or contact along their inner edges and are provided with an enlarged seams to allow radially inward movement. Consequently, because of the enlarged seams, clearance is provided for the arms to deflect radially outwardly or inwardly relative to a lengthwise axis of the valve actuator without the inner edges delimiting the radially inward deflection.

In an example, each of the two enlarged seams of the two pairs of partial bridges has a width or a gap. The width or gap can be measured between the adjacent inner edges of two adjacent partial bridges. In an example, the dimension of the width or gap of each enlarged seam is smaller or narrower than a width of a distal wall of the needle guard, or at least smaller than the dimension of the arms of the needle guard at the elbows. This arrangement allows the two bridges of the present valve actuator or opener to impede proximal movement of the needle guard in the ready position and during retraction of the needle following successful venipuncture, even with the enlarged seams.

To couple the needle guard to the valve actuator so that the two elbows of the needle guard are located distal of the distal edges of the partial stabilizers, at the distal cavity opening or chamber of the valve actuator without compressing or bending the needle guard, the two actuator arms can be deflected outwardly relative to the longitudinal axis of the valve actuator from a first position to a second position in which the proximal ends are spaced apart further away to widen the enlarged seams of the two pairs of partial bridges. The now further widened enlarged seams allow the valve actuator to be placed over the needle guard, while the needle guard is mounted on a needle, without deflecting or compressing the needle guard to fit through the opening defined by the two actuator arms and the four partial bridges.

An interior surface of the catheter hub can be provided with a couple of bumps or projections, which can be located and oriented inside the catheter hub to align with and occupy the spaces at the two enlarged seams when the valve actuator is located inside the catheter hub during assembly.

Two projections can be equally spaced within the interior of the catheter hub. The two projections can be provided to prevent rotation of the valve actuator about the lengthwise axis of the catheter hub. The two projections can also be provided to limit deflection of the two arms inwardly towards one another by occupying most if not all of the spaces defined by the two enlarged seams. For example, a male Luer tip inserted into the catheter hub to advance the valve actuator in the distal direction to open a valve can cause the two actuator arms to deflect inwardly towards one another if not for the projections. Thus, the projections can operate to limit the inward deflection of the two valve opener arms. In an alternative embodiment, only one projection is incorporated inside the catheter hub and the one projection can still limit radially inward deflection of the two valve opener arms.

During assembly, an NGA sub-assembly, or sub-assembly for short, may first be formed prior to mounting the sub-assembly to a catheter hub. The NGA sub-assembly can include a needle, a guard, and an actuator. The needle can be attached to a needle hub. The NGA sub-assembly with the valve opener and a needle guard can be formed prior to assembling the sub-assembly to a catheter hub, such as prior to sliding the needle with the valve opener and needle guard into the catheter hub and the catheter tube.

The NGA sub-assembly can be installed or assembled to a catheter hub having a catheter tube. The valve actuator can be located proximally of a valve and the actuator head can be in a space defined by a shroud of the valve. In other examples, the valve can be without a shroud. In the installed configuration, two projections can be located proximally of the two elbows of the needle guard. The needle guard can be located between two actuator arms. The two projections can generally align with the distal edges of the four partial bridges.

Two projections can define a minimum inside diameter inside the catheter hub. Thus, as the NGA sub-assembly is inserted into the catheter hub during assembly, the two arms of the needle guard can undergo some deflection at the elbows in order to fit within the minimum inside diameter at the two projections. However, assembly of the needle guard to the valve opener when forming the NGA sub-assembly can be carried out without compressing the two arms to the same extent, or any, to pass through the opening defined by the bridges and valve opener arms.

In another example, two actuator arms of a valve actuator can be fabricated in a normal extended position from the actuator head and are connected to one another with a single bridge or stabilizer element. The bridge can be without a seam and does not readily allow the two arms to deflect inwardly or outwardly relatively to the lengthwise axis of the valve actuator.

With only a single stabilizer element, the valve actuator does not have a restricted or defined opening defined by two actuator legs and two stabilizer elements. Said differently, the valve actuator of the present embodiment does not have a fully enclosed structure along the radial direction at the single bridge. Accordingly, when the valve actuator of the present embodiment is coupled to a needle guard to form an NGA sub-assembly, the valve actuator can be manipulated back-and-forth and up-and-down to move over the needle guard without compressing or distorting the needle guard to do so, or only some minimum amount to do so, due at least to the open bottom of the valve actuator not incorporating a second bridge or stabilizer element.

Where a sub-assembly incorporates a valve opener with only a single bridge, a catheter having a single projection can be used with the sub-assembly. A projection is not required inside the catheter hub on the side with the bridge. In the installed configuration, the single projection inside the catheter hub is located proximally of one of the two elbows of the needle guard, on the side without the stabilizer element or bridge. The projection is generally aligned with the distal edge of the bridge, and is proximally of the elbow of the needle guard. The stabilizer element or bridge and the projection both prevent the needle guard from displacing in the proximal direction in the ready position and during retraction of the needle following successful venipuncture.

The single projection and the single bridge define a minimum inside diameter in the catheter hub. Thus, as the NGA sub-assembly is inserted into the catheter hub, the two arms of the needle guard can undergo some deflection at the elbows in order to fit within the minimum inside diameter at the projection and the bridge.

In yet another example, a valve opener can include two stabilizer elements connecting the two actuator arms. The two stabilizer elements can be without any seam. Further, the contour of the two stabilizer elements can be generally flat or planar. Said differently, each stabilizer element can comprise a planar surface rather than an arcuate contour. Further, the distal edge and the proximal edge of each stabilizer element can be generally parallel to one another and to the distal and proximal edges of the other stabilizer element, or the second stabilizer element.

In some example, a bridge of a valve opener can have a distal edge and a proximal edge and wherein the distal edge is orthogonal to a lengthwise axis of the valve actuator or angled to the lengthwise axis of the valve actuator. In some examples, the entire distal edge is either orthogonal to the lengthwise axis of the valve actuator or angled to the lengthwise axis of the valve actuator.

In some examples the distal edge and the proximal edge are parallel to one another. In still other examples, the distal edge and the proximal edge can be angled to one another, such as converge or diverge.

In use, the valve actuator can be assembled or coupled with a needle guard and then the combination mounted onto a needle before a change in profile is formed with the needle. Thus, an NGA sub-assembly can be formed with the present valve actuator prior to placing the valve actuator inside a catheter hub. Optionally, the present valve actuator can be placed inside a catheter hub prior to positioning a needle with a needle guard into the catheter hub and using the nose section of the needle hub to push the needle guard in through the opening defined by the two stabilizer elements and the two valve opener arms.

In still another example, a valve opener can include two stabilizer elements connecting the two actuator arms together and wherein the two stabilizer elements do not incorporate any seam. Further, the contour of the two stabilizer elements can be generally arcuate rather than planar. Said differently, each stabilizer element of the present embodiment comprises an arcuate or curved surface that curves away from the lengthwise axis of the valve opener.

Methods of making the catheter assemblies and their components described elsewhere herein are within the scope of the present disclosure.

Methods of making the NGA sub-assemblies and their components described elsewhere herein are within the scope of the present disclosure

### BRIEF DESCRIPTION OF THE DRAWINGS

These and other features and advantages of the present devices, systems, and methods will become appreciated as the same become better understood with reference to the specification, claims and appended drawings wherein:
FIG. 1 is a side view of a catheter assembly in a ready position in which the needle tip extends out a distal end of a catheter tube;
FIG. 2 is an exploded isometric view of the catheter assembly of FIG. 1;
FIG. 3 is a schematic cross-sectional side view of the catheter assembly of FIG. 1;
FIG. 4 is a schematic partial cross-sectional side view of the catheter assembly of FIG. 1 in a transition position or state in which the needle is in the process of being removed from the catheter tube and the catheter hub, such as following successful venipuncture;
FIG. 5 is a schematic cross-sectional side view of the catheter assembly of FIG. 1 in which the needle has completely separated from the catheter hub and the valve opener is available for advancing against the valve to open the valve;
FIG. 6 is a schematic cross-sectional side view of the catheter assembly of FIG. 1 in which the catheter hub is now connected with a male Luer and the valve actuator is advanced by the male Luer to open the valve;
FIG. 7 is a schematic cross-sectional side view of a catheter hub in accordance with aspects of the present disclosure;
FIG. 8A is a schematic cross-sectional side view of a valve embodiment with an extended flange in accordance with aspects of the present disclosure;
FIG. 8B is a schematic cross-sectional side view of another embodiment of a valve with an extended flange on opposite sides of the valve disc in accordance with aspects of the present disclosure;
FIG. 8C is an isometric view of a valve, which can be the valve of FIGs. 8A or 8B;
FIGs. 9A is a schematic cross-sectional side view of another embodiment of a valve in accordance with aspects of the present disclosure;
FIG. 9B is an isometric view of the valve of FIG. 9A;
FIGs. 10A-10D show different views of yet another embodiment of a valve actuator in accordance with aspects of the present disclosure;
FIG. 10E shows a sub-assembly for use with a catheter hub having a catheter tube, the sub-assembly having a needle, a valve opener, a needle guard, and a needle hub;
FIGs. 10F(a) and (b) show different assembly views of a catheter assembly;
FIGs. 11A-11C show different views of yet another embodiment of a valve actuator in accordance with aspects of the present disclosure;
FIGs. 11D and 11E show different views of a catheter hub having one or more internal projections;
FIG. 11F is a cross-sectional side view showing a sub-assembly having a needle, a valve opener, a needle guard, and a needle hub assembled to a catheter hub having a catheter tube;
FIGs. 12A-12C show different views of yet another embodiment of a valve actuator in accordance with aspects of the present disclosure;
FIG. 12D shows a sub-assembly for use with a catheter hub having a catheter tube, the sub-assembly having a needle, a valve opener, a needle guard, and a needle hub;
FIG. 12E is a cross-sectional side view showing a sub-assembly having a needle, a valve opener, a needle guard, and a needle hub assembled to a catheter hub having a catheter tube;
FIGs. 13A and 13B show different views of yet another embodiment of a valve actuator in accordance with aspects of the present disclosure; and
FIGs. 14A and 14B show different views of yet another embodiment of a valve actuator in accordance with aspects of the present disclosure.

### DETAILED DESCRIPTION

The detailed description set forth below in connection with the appended drawings is intended as a description of the presently preferred embodiments of catheter assemblies with control valves provided in accordance with aspects of the present devices, systems, and methods and is not intended to represent the only forms in which the present devices, systems, and methods may be constructed or utilized. The description sets forth the features and the steps for constructing and using the embodiments of the present devices, systems, and methods in connection with the illustrated embodiments. It is to be understood, however, that the same or equivalent functions and structures may be accomplished by different embodiments that are also intended to be encompassed within the scope of the present disclosure. As denoted elsewhere herein, like element numbers are intended to indicate like or similar elements or features.

With reference now to FIG. 1, a catheter assembly 100, which may more broadly be referred to as a needle assembly, a safety needle assembly, or a needle device, having a blood control system implemented therewith is shown, which comprises a catheter hub 102 with a catheter tube 104 attached to a hub body 103 and a needle hub 106 with a needle 108 having a needle shaft 109 (FIG. 2) extending through the catheter hub 102 and the catheter tube 104 with a needle tip 110 extending out a distal end or distal opening 112 of the catheter tube 104 in a ready to use position or ready position. The needle assembly is sized and shaped for intravenous access or peripheral access. In the ready position, the catheter assembly 100 is ready for use, such as to perform a venipuncture or intravenous access. Sometimes the ready position first requires removing a protective cap (not shown) from the catheter assembly or needle assembly 100.

FIGs. 2 and 3 show an exploded view and a schematic cross-sectional view, respectively, of the catheter assembly 100 of FIG. 1. A flashback plug 114 can be provided at the proximal end 118 of the needle hub 106 to allow air to vent but stops blood from spilling out the proximal opening of the needle hub when blood enters the flashback chamber 116 during primary flashback. Alternatively, a male medical implement such as a syringe can be attached to the proximal end 118 of the needle hub 106.

The needle hub 106 can further comprise a shoulder, a tab, or a surface feature 120 that physically contacts the catheter hub 102, such as the proximal end surface 122 of the catheter hub 102, to axially register the catheter and needle hubs 102, 106 to set the length of the needle tip 110 projecting out of the distal opening 112 of the catheter tube 104.

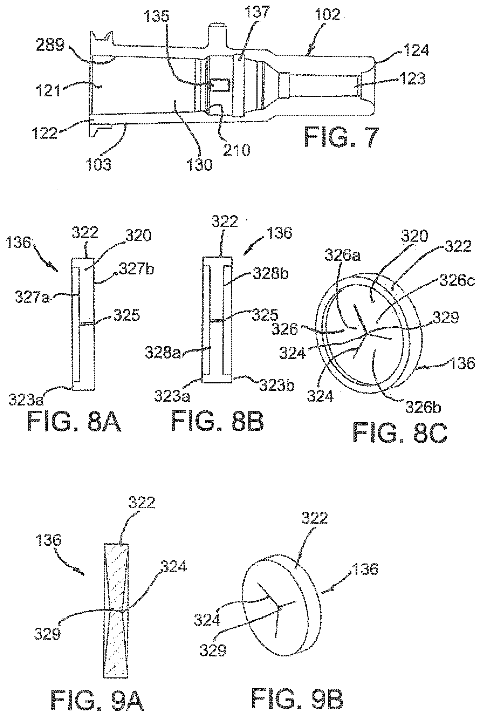

Jumping ahead to FIG. 7, a schematic cross-sectional side view of the catheter hub 102 of FIG. 1 is shown without the catheter tube. The catheter hub 102 can have a proximal opening 121 and a perimeter defining the proximal opening at a proximal end surface 122. The hub body 103 has an interior surface 288 that defines an interior cavity 130 and has a distal opening 123 at a distal end surface 124 opposite the proximal end surface 122. The catheter hub 102 can be a standard catheter hub, can include a pair of wings, can be a ported catheter hub, or can be an integrated catheter hub with a tubing extending from a side tubing port. A needleless valve can attach to the opposite end of the tubing.

With further reference to FIG. 2 and continued reference to FIG. 7, a tip protector or needle guard 132, a valve actuator or valve opener 134, a valve 136 for occluding fluid flow, and a bushing 138 can be provided inside the interior cavity 130 (FIG. 6) of the catheter hub 102. As further discussed below, the valve opener 134 is configured to slide into and push open the valve 136 when advanced by a male Luer tip inserted into the proximal opening 121 of the catheter hub (102), after successful venipuncture. The needle shaft 109 of the needle 108 can extend through the needle guard 132, the valve opener 134, a valve opening 325 defined by one or more slits of the valve 136, the bushing 138, and the catheter tube 104 in the ready to use position. The one or more flaps of the valve 136 may be elastically opened so that the needle shaft 109 can pass through the valve opening 325, as further discussed below in reference to FIGs 8A-8C and FIGs. 9A and 9B.

Referring again to FIG. 7, the catheter hub 102 can include a slot or groove 135 or a pair of slots or grooves 135 of desired depth, width, and length formed in or on the interior surface 288 of the interior cavity 130 to guide axial displacement of the valve opener 134 when the valve opener is actuated, such as when advanced by a syringe tip or a male Luer tip of an IV administration set or an extension set. For example, the valve opener 134 can have tabs that are located within the one or more grooves 135 to align within the interior of the catheter hub. The slots 135 can be spaced from one another, such as equally spaced along the inner circumference of the catheter hub 102. The slots 135 can each have a rectangular opening and a length of the opening orientated parallel to the lengthwise axis of the catheter hub. In other examples, the slots can have other shaped openings. In other examples, there can be only one slot 135 or more than two slots 135. The number of slots can depend on structures or features incorporated with the valve opener 134, which can interact or cooperate with the corresponding number of slots to align or retain the valve opener within the catheter hub.

A groove 137 may be formed inside the interior cavity 130 of the catheter hub to function as a valve seat, such as to surround and secure an outer perimeter of the valve 136 inside the catheter hub. The groove 137 can be circumferential or ring shape and can be located distally of the one or more slots 135. The groove and the slots can be formed by recessing the interior surface 289 of the catheter hub 102at selected locations, such as by thinning out selected interior locations of the catheter hub. In other examples, slots and grooves can be formed by raising the interior surface of the catheter hub at selected locations to cooperate with corresponding structures on the valve opener.

A guard engagement section 210 can be located proximally of the slots 135 and can be provided so that the needle guard 132, such as an elbow of the needle guard, can bear, rest, or contact therewith in the ready to use position and during needle withdrawal prior to activation of the needle guard 132 as further discussed below. The guard engagement section 210 can comprise an internal projection, such as a first inside diameter section located adjacent a second inside diameter section, which is larger than the first inside diameter section. The internal projection can be continuous or non-continuous, such as made from several spaced sections. The order or location of the groove 137, the slots 135, and the guard engagement section 210 can vary to accommodate differently shaped valves, valve openers, and needle guards. The proximal end 122 of the catheter hub 102 can be sized with a female Luer taper to receive a male Luer tip. The bushing 138 can be configured to retain the catheter tube 104 to the catheter hub 102. In one example, the bushing 138 can wedge the proximal end of the catheter tube 104 against the interior wall surfaces of the catheter hub 102 to retain the catheter tube 104 to the catheter hub 102.

The needle guard 132 may embody any number of prior art guards, tip protectors, or safety clips configured for blocking or covering the needle tip 110 of the needle 108. In the exemplary embodiment shown, the needle guard 132 can embody one of the guards shown in US Pat. No. 6,616,630. In one example, as shown in FIGs. 2 and 4, the needle guard 132 can have a proximal wall 280 having a proximally facing wall surface and a distally facing wall surface with a perimeter defining an opening and two resilient arms 288, 290. The needle 108 can comprise a change in profile 144, which can be an attachment or sleeve, a crimp, or a bulge, for engaging the perimeter on the proximal wall of the needle guard 132 to retract the needle guard 132 in the proximal direction out of the catheter hub 102 following successful venipuncture, as further discussed below.

In the ready to use position, the proximal wall 280 may be in contact with a distal end 119 of the needle hub 106, such as to a nose section, a tab, or a fin at a distal end of the needle hub. The two arms 288, 290 of the guard can intersect along a side view as described in US Pat. No. 6,616,630 and shown in FIGs. 2 and 4, or they can run along different sides of the needle and do not intersect along a side view. In one example, one arm can be longer than the other arm. Each arm 288, 290 can also include different sections of different arm widths, including a first arm section of a first width and a second arm section of a second width, which is smaller than the first width. The two arms 288, 290 can originate from different ends of the proximal wall 280 and can cross one another at their respective second arm sections. Thus, when viewed from a side along the lengthwise direction of the needle guard 132, the two arms intersect one another. When used with a needle 108, the two arms 288, 290 intersect one another when in a ready to use position and when in the protective position.

In an alternative embodiment, the two arms 288, 290 originate from different ends of the proximal wall and extend in a distal direction without crossing one another. Thus, the two arms 288, 290 can also have essentially the same arm width along the length of each respective arm. The needle guard 132 may be folded from a stamped metal sheet to form the guard as shown. Ribs 296 may be formed on the arms, the proximal wall, and/or distal walls of the arms to increase structurally rigidity. In some examples, the needle guard can be assembled from different components or parts and can include both metal and non-metal parts.

A distal wall 300, 302 can be provided at an end of each arm 288, 290. The distal walls 300, 302 on the two arms can overlap one another along an axial direction of the needle guard 132 when the arms close over the needle tip by utilizing different arm lengths and/or angling one of the walls at intersections with the resilient arms 288, 290 so that they are positioned serially along the length of the needle. The intersection between a distal wall and the elongated portion of an arm may be referred to as an elbow or a diagonal diameter 304 of the needle guard when referring to the two elbows on the two arms. In an example, an intersection or elbow 304 between the distal wall 300 and the elongated portion of the resilient arm 288 can engage the guard engagement section 210 inside the catheter hub 102 to secure the needle guard 132 to the catheter hub 102 in the ready to use position, as shown in FIG. 3, and during the process of removing the needle 108 from the catheter hub 102, as shown in FIG. 4. Thus, the guard engagement section 210 can prevent the needle guard 132 from moving proximally by presenting a physical barrier to the one or two elbows 304 before the needle guard 132 is activated. That is, in the ready to use position, the cross-sectional dimension or diagonal diameter profile at the two elbows is larger than an inside diameter at the guard engagement section 210. Once the needle guard is activated to cover or block the needle tip 110 in the protected position, the two elbows move closer together to decrease the diagonal diameter profile, which then allows the two elbows to move proximally through the internal diameter defined by the guard engagement section 210 of the catheter hub. In some examples, the two distal walls of the needle guard can be called or be considered part of the two arms.

The needle guard arms 288, 290 can be biased outwardly by the needle shaft 109 in the ready position, as shown in FIG. 4. When the arms are biased outwardly, the radial profile of the needle guard increases. For example, the cross-sectional dimension measured between the two elbows of the two arms can increase by moving away from each other when a needle is located between the two arms and biases the two arms outwardly. When the arms are not biased outwardly, such as when the needle is no longer located between the two distal walls of the two arms, the radial profile of the needle guard decreases, compared to when the arms are biased outwardly.

The arms can be biased outwardly so that the intersections 304 of the two arms engage the guard engagement section 210 of the catheter hub 102 to retain the guard within the catheter hub in the ready to use position and during retraction of the needle following successful venipuncture. Said differently, the needle shaft 109 can apply an outward radial force on the lips or end surfaces 306 formed at the free ends of the two distal walls 300, 302 so as to urge the distal walls 300, 302, and the arms, outwardly to then engage the intersections 304 with the guard engagement section 210 of the catheter hub, or at least maintain the intersections 304 distal of the engagement section 210. In some examples, the engagement section 210 can be omitted or the intersections 304 can be situated inside the catheter hub without engaging the engagement section 210 of the catheter hub, as further discussed below. In still other examples, only one intersection of one of the arms is biased outwardly into engagement with the engagement section 210 of the catheter hub. In still yet other examples, a valve opener is located inside the catheter hub and the needle guard interacts with the valve opener to prevent the needle guard from moving in the proximal direction during retraction of the needle and in the ready to use position, until the needle tip moves proximally of the one or two distal walls.

Referring now to FIG. 4, the lips 306 formed at the free ends of the distal walls 300, 302 are contacted by and are biased outwardly by the needle shaft 109. The lips 306 formed at the free ends of the distal walls 300, 302 can be rounded or curved to decrease drag on the needle shaft 109 during needle withdrawal. Alternatively, the lips 306 may be straight or not provided with the needle guard 132. In other examples, the distal walls are biased outwardly by the valve opener and the lips 306 are spaced from the needle shaft. In these examples, there is no drag generated between the needle shaft and the distal walls of the needle guard.

In an example, the needle guard 132 can be positioned inside a valve opener 134 or between two spaced apart structures of the valve opener so that no part of the free ends of the arms, at the ends of the two distal walls 300, 302, contact or are biased by the valve opener, as further discussed below. In other examples, as further discussed below, the needle guard is biased outwardly by the needle so that the distance between the two elbows of the needle guard is increased and the two elbows are prevented from moving in the proximal direction in the ready position and during retraction of the needle by one or more bridges or bridge sections of the valve opener.

Referring back to FIGs. 3 and 7, the guard engagement section 210 can be formed in the interior cavity 130 of the catheter hub 102 to hold the needle guard 132 in the ready to use position and during needle withdrawal, such as when the arms of the needle guard 132 are biased outwardly by the needle shaft 109. The guard engagement section 210 can extend radially inwardly into the interior cavity of the catheter hub 102 to form a minimum diameter inside the catheter hub. The guard engagement section 210 can have an interference overlap with the diagonal diameter of the needle guard 132, such as the two intersections 304 of the two arms, to ensure the needle guard 132 is secured in place inside the catheter hub in the ready to use position and during retraction of the needle. A tapered or slanted surface at the two intersections 304 can decrease the pulling force required to disengage the needle guard 132 from the guard engagement section 210 of the catheter hub 102.

The needle guard 132 can be assembled inside the interior cavity 130 of the catheter hub 102 by pushing the proximal wall 280 of the needle guard 132 with the distal end of the needle hub 102 as the needle hub 102 with needle 108 is inserted into the catheter hub 102. In the ready to use position, the needle guard 132 can extend at least partially into a gap of a valve opener with the proximal section of the needle guard 132, such as the proximal wall 280, located outside or proximal of the valve opener 134. That is, the proximal wall 280 of the needle guard 132 can be located proximally of the proximal end surface or surfaces 151 of the valve opener 134.

In another example, the proximal wall 280 of the needle guard 132 can be flush or coplanar with the proximal end surfaces 151 of the valve opener 134. In yet another example, the proximal wall 280 of the needle guard 132 can be located distally of the proximal most surfaces of the valve opener 134. In embodiments in which the valve opener 134 biases the two arms outwardly, the distal walls of the two arms either do not fit within the gap 154 of the valve opener or the valve opener is provided with seat clips or arm seats to support the two distal walls, as further discussed below.

When the needle tip 110 is pulled in a proximal direction into the needle guard 132 following successful venipuncture, the change in profile 144 on the needle 108 engages a perimeter 282 defining an opening 284 (FIG. 2) on the proximal wall 280 of the needle guard 132, as previously described. As the needle is continued to be moved in the proximal direction, the arms 288, 290 of the needle guard 132 may simultaneously or soon thereafter, collapse to their protective position to block accidental contact with the needle tip 110 when clear of the needle shaft 109 or the clip seats of the valve opener 134. The same working can also be achieved by one of the one arm needle guards described in U.S Pat. No. 6,616,630, which runs along a side of the needle shaft instead of crossing the needle as shown in some of the embodiments of the '630 patent.

With reference now to FIG. 5, after the needle hub 106 and needle 108 are withdrawn from the catheter hub 102 and the needle tip 110 is secured by the needle guard 132, the opening 325, or valve opening, of the valve 136 can reseal upon itself to prevent or limit blood or other fluid from passing through the valve opening 325. That is, after withdrawal of the needle shaft 109 from the valve opening 325, the potential elastic energy stored in the valve 136 generated from the needle shaft 109 deflecting the flaps of the valve 136 surrounding the valve opening 325 is released and the flaps return to a less deflected or closed position to reseal the valve opening 325. Thus, FIG. 5 shows the needle 108 completely removed from the catheter hub 102 and the valve opener 134 ready to be pushed by a male Luer tip inserted into the proximal opening of the catheter hub and pushing distally against the proximal end surfaces 151 of the valve opener 134 to advance the valve opener against the valve 136 to open the valve 136.

The catheter hub 102 can be designed such that an engagement distance "A" between the proximal end 122 of the catheter hub 102 and the proximal end surfaces 151 of the valve opener 134 may be less than or equal to the minimum length "B" of engagement of a male medical implement, such as a syringe tip or a male Luer fitting, into the catheter hub 102. This can ensure that in the event of a connection of a male lock fitting with the catheter hub, the male lock fitting would introduce a longitudinal force or axial force along the lengthwise axis of the catheter hub to the valve opener 134 and allow a longitudinal displacement of the valve opener 134 towards the proximal end of the valve 136 to open the valve 136. In one example, the engagement distance is per ISO standard, as presently established or established in the future.

The catheter hub 102 can also be designed such that the minimum depth "B" of the interior cavity of the catheter hub 102 from the proximal end 122 of the catheter hub 102 may be greater than or equal to the minimum length of the male lock fitting to ensure that in the event of a connection of a male lock fitting, the male lock fitting would have sufficient surface contact area with the female Luer taper at the proximal opening 121 of the catheter hub 102.

FIG. 6 shows a male medical implement 220, such as a male Luer lock or a syringe tip, inserted into the interior cavity 130 of the catheter hub 102 through the proximal opening 121 at the proximal end 122 of the catheter hub 102 to displace the valve opener 134 with the distal end 125 of the tip in a distal direction to press against the valve 136 to open the valve 136. In one example, when a male Luer lock fitting is attached from the proximal end 122 of the catheter hub 102, the male Luer lock fitting will contact the valve opener arms 152. As the lock fitting is threaded with the exterior threads at the proximal end 122 of the catheter hub 102, the male fitting pushes against the proximal end surfaces 151 of the valve opener arms 152 distally until the head 150 of the valve opener pushes the valve 136 open.

When the valve 136 opens, fluid can pass through the valve opening 325 of the valve. When the lock fitting of the male medical implement 220 is subsequently removed, the valve 136 can elastically recover back to reseal the valve opening 325 (FIGs. 8A, 8B). As the valve 136 reseals itself, the valve 136 can stop or limit flow through the opening while simultaneously push the valve opener 134 proximally back to its ready to use position or its proximal position of FIG. 5 thereby allowing multiple use of the blood control system. In other examples, the valve and valve opener can be configured as a single use valve. That is, when the valve opener is pushed into the valve, the two remain engaged even after removal of the male Luer tip.

In an example, the actuator head or nose end 150 of the valve opener has a frusto-conical shape so as to readily deflect the flaps of the valve in the distal direction to open the valve. The valve flaps of the valve 136 may be made sufficiently resilient, such as by making the flaps thicker, so as to return to its closed position after the male Luer tip 50 is removed and an axial load is no longer applied to the valve actuator. In another example, an elastic element, such as a resilient gasket or a spring, may be incorporated to assist with closing the flaps on the valve. The frusto-conical shape of the actuator head 150, when incorporated, can retain a proximally directed force vector of the flap against the conical surface, which can push the actuator back to its starting position until the flaps are closed again and flow is stopped. Various embodiments of the valve opener 134 and various embodiments of the valve 136, which may be implemented in the blood control system, are further discussed below.

With reference now to FIGs. 8A and 8B, a cross-sectional side view and an isometric view of an exemplary valve 136 provided in accordance with aspects of the present disclosure are shown. The valve 136 may be usable with the catheter assemblies and hubs with a female Luer described elsewhere herein. The valve 136 can be called or considered a disc valve. The disc valve can optionally include one or more flanges extending therefrom, as further discussed below. The valve 136 is shown with a valve body 320 having a width measured from one edge to another edge of an outer perimeter or outer diameter 322 of the valve body 320. The valve body 320 has a thickness, which is the dimension that extends orthogonal to the width from a first surface 327a to a second surface 327b. The first and second surfaces 327a, 327b can be proximally and distally facing surfaces depending on the direction of use of the valve. The valve 136 can be seated in a valve seating area or valve groove 137 (FIG. 7) provided in the interior cavity of the catheter hub 102. The valve is configured to be located distally of a valve opener 134 to form a seal in the interior cavity and to be opened by the valve opener. In some examples, the valve can be placed between two different hub sections of a catheter hub and held therebetween.

The valve groove 137 can be a radial undercut formed in the interior cavity of the catheter hub 102 with a depth in the range between about 0 mm to about 1 mm deep, or recessed, to allow proper seating. The depth range can have a tighter tolerance of about 0 mm to about 0.3 mm. A seal is provided between the interior of the catheter hub 102 and the outer surfaces of the valve 136, such as with the outer perimeter 322. In one example, a slight compressive force can be applied by the interior of the catheter hub against the perimeter of the valve to ensure sealing. Alternatively or in addition, the outer edges of the first and second surfaces 327a, 327b can seal against the surfaces of the valve groove 137 to provide a seal with the catheter hub. In an example, the profile of the valve groove 137 as well as any profile internally of the catheter hub, such as slots or projection(s), can be contoured with radiuses to minimize smearing of material during the molding process of the catheter hub. In other examples, such as when using different thermoplastic or elastomeric material to form the valve, the tolerance can vary.

A valve opening 325 can extend through the thickness of the valve body 320 from the first surface 327a to the opposite second surface 327b. The thickness of the valve body 320 can be uniform or can vary. For example, the thickness between the first and second surface 327a, 327b can vary to vary the thickness of the valve disc and the valve flaps. The thinner the valve 136, such as the thinner the valve flaps, the less resistance the valve 136 is to opening, and therefore less force is required to open the valve 136. The thicker the valve, the more elastic energy is able to be stored to enable the valve to return to its original pre-opened shape upon elastic deformation, and thus allowing multiple access of the valve opening and closing. In one example, the thickness of the valve is in the range of about 0.3 mm to about 1.5 mm. However, the thickness can vary and can include other ranges and varying thicknesses to control aspects of the flaps.

A first flange 323a can extend axially from the outer perimeter or outer diameter 322 of the valve body 320 to allow better seating of the valve in the catheter hub 102. The first flange 323a can be oriented in the catheter hub to extend proximally as shown in FIG. 1 or distally, which depends on the orientation of the valve when situated inside the catheter hub. The first flange 323a and the valve body 320 can cooperatively define a first circular cavity 328a concentric with the valve body 320 and locally reducing the thickness of the valve 136 at the first circular cavity 328a.

The valve opening 325 is shown with or defined by three slits 324 extending radially from a center of the valve 136 and formed approximately 120 degrees apart, thereby forming a plurality of flaps 326 including a first flap 326a, a second flap 326b, and a third flap 326c. That is, the three slits 324 can intersect at a single central point 329 coinciding with the axis of the valve 136 and forming three flaps. The length of the slits can vary. In one example, the slits extend to the first flange 323a but can extend a shorter length as noted by the available length variation. The first flap 326a, the second flap 326b, and the third flap 326c can be deflected to open a flow path 226 through the valve body 320. The fluid flow path 226 is provided when the three flaps are deflected by the valve opener. In an example, the flaps 326a, 326b, 326c near the central point 329 expand radially towards the perimeter 322 and in the distal direction when deflected by the valve opener 134. That is, the first flap 326a, the second flap 326b, and the third flap 326c can be deflected by pushing the valve 136 with one of the valve openers 134 described herein on a proximal side of the valve 136, as discussed further below.

Alternatively, the valve opening 325 can be formed by a single slit 324, such as having at least one slit, formed through the thickness of the valve body 320 and defining a first flap and a second flap, which can also be deflected to open a flow path through the valve body 320 by pushing the valve 136 with a valve opener 134 on one side of the valve.

In an example, the valve opening 325 may also include reliefs embodying two short through cuts at each end of the slit forming a V-shaped relief. The reliefs can provide clearance for the flaps to enable them to deflect more readily when pushed open by the valve opener 134. Less preferably, a single short through cut may be incorporated at each end of the slit.

Referring to FIG. 8B, a second flange 323b can extend axially from the outer perimeter of the valve 136 opposite the first flange 323a thereby cooperatively forming a second circular cavity 328b concentric with the valve body 320 and further reducing the thickness of the valve 136 at the first and second circular cavities 328a, 328b.

FIGs. 9A and 90B are a cross-sectional side view and an isometric view, respectively, of an exemplary valve 136 provided in accordance with further aspects of the present disclosure, which may be usable with the catheter assemblies and hubs with a female Luer described elsewhere herein. The present valve 136 is similar to the valve of FIGs. 8A-8C with a few exceptions. In the present embodiment, the valve 136 has a circular valve body with a varying thickness that increases from approximately a central point 329 of the valve 136 towards the outer perimeter 322 of the valve 136. The thickness can vary linearly with a constant slope or can have a complex slope. The valve 136 has an opening 325 with three slits 324 provided through the thickness of the valve body 320 to form three flaps 326a, 326b, 326c. The three slits 324 can intersect at a single central point 329 coinciding with the axis of the valve 136. The flaps can be deflected to open a flow path 226 through the valve body 320. The first, second and third flaps 326a, 326b, 326c can be deflected by pushing the valve 136 with a valve opener to deflect the flaps radially and axially. A fluid flow path 226 can be provided when the three flaps are deflected. In an example, the flaps 326a, 326b, 326c near the central point 329 expand radially towards the outer perimeter 322 and in the distal direction when deflected by the valve opener 134 pressing against the proximally facing surface of the valve 136.

With reference again to FIG. 3, the valve 136 can be located inside the catheter hub 102 just distal of the head 150 of the valve opener 134. The valve 136 can comprise an outer perimeter that can be seated in a valve groove 137 (FIG. 7) to fix the outer perimeter of the valve 136 between the valve opener 134 and the bushing. Alternatively, the outer perimeter of the valve 136 can be fixed to the interior cavity of the catheter hub 102 between the valve opener 134 and the bushing 138 by interference fit, adhesive, or other securing means.

FIG. 5 shows the needle 108 completely removed from the catheter hub 102 with the needle guard 132 (FIG. 3) covering the needle tip 110 in a protective position, which is not shown but is understood in the relevant art. To get to this position, the needle device 100 starts at the position of FIG. 3, which may first require removal of a disposable protective cap, moves to a transition position of FIG. 4 wherein the needle slides proximally relative to the catheter tube and the catheter hub, and then continuing to move the needle until the needle tip 110 moves proximally of two distal walls 300, 302, one on each end of the resilient arms 288, 290 of the needle guard 132. The distal walls can be considered part of the arms on the needle guard and are specifically called out so as to identify the structure and function in relations to the other components and how the disclosed needle assemblies operate.

Where the needle guard 132 has only one distal wall and/or one arm, the process is similar but the needle tip only has to move proximally of the one distal wall to cause the needle guard to activate. As the two distal walls and hence the two resilient arms 288, 290 are no longer biased outwardly by the needle 10 or valve opener 134, the two arms 288, 290 move radially to decrease the guard's radial profile and to disengage from the guard engagement section 210 of the catheter hub 102. In an example, two elbows dislodge from the guard engagement section 210 of the catheter hub when the needle tip moves proximally of the two distal walls. Alternatively, the one arm and one distal wall can disengage from the one guard engagement section 210.

Where the arms of the needle guard are biased outwardly by the clip seats of the valve opener instead of the needle and the needle tip moves proximally of the two distal walls, the arms remain biased until the change in profile on the needle contacts the perimeter on the proximal wall of the needle guard. Further movement of the needle from the point the change in profile engages the perimeter on moves the proximal wall, and hence the entire needle guard, in the proximal direction until the distal walls on the arms separate from the clip seats on the valve opener, or from the valve opener arms. At such time, the distal walls of the needle guard move radially inwardly to block the distal tip of the needle in a protective position.

As the needle continues to move in the proximal direction and the change in profile 144 on the needle engages the perimeter 282 on the proximal wall of the needle guard 132 (FIG. 4), the needle guard 132 moves proximally with the needle 108. Alternatively the needle guard 132 can clamp onto the needle shaft 109 and be removed from the catheter hub 102 as a unit with the needle without utilizing a needle crimp. Note that in the protective position in which the needle guard 132 covers the needle tip 110, the valve 136 and the valve opener 134 remain inside the interior cavity of the catheter hub 102. Thus, the valve 136 and the valve opener 134 are located inside the catheter hub 102 in both the ready position of the needle and the protective position of the needle 108. Viewed from another perspective, the valve 136 and the valve opener 134 are located inside the catheter hub 102 in both the ready to use position of the catheter assembly 100, in which the needle tip projects out a distal opening 112 (FIG. 1) of the catheter tube 104, and a protective position of the catheter assembly 100, in which the needle 108 is removed from the catheter hub 102 and the needle tip 110 is covered by a needle guard 132.

With reference now to FIG. 6, the catheter hub 102 is shown with a male medical implement 220 positioned in the proximal opening thereof. The male medical implement or instrument 220 can be a male Luer, a syringe tip, an IV set connector, or other male tip having a Luer taper. For discussion purposes, the male medical implement has a male tip 50. For example, the male medical implement can be connected to an IV tubing, which is connected to an IV fluid source for fluid delivery through the male medical implement 220, the catheter hub 102, and the catheter tubing 104 to deliver fluid therapy to a patient.

When initially inserting the male medical implement 220, herein male tip 50, into the proximal opening of the catheter hub 102, the male tip initially contacts the two valve opener arms 152 on the valve opener 134 to advance a distally directed force onto the two valve opener arms 152 to open the valve 136. The proximal end surfaces 151 of the valve opener arms 152 can provide a contact surface for the distal end surface 125 of the male medical instrument 220, as previously discussed. The valve opener arms can also be designed to contact the inside wall of the catheter hub at a tangential point. In this way, the valve opener arms 152 are stable and can resist being deflected outwards. This arrangement can avoid relatively thin valve opener arms from wedging between the male medical instrument 220 and the inside wall of the catheter hub 102. The distally directed force moves the valve opener 134 in the distal direction until the geometries of the male tip 220 and the proximal opening 121 of the catheter hub 102 stop further distal advancement of the male tip 50. In an example, a female Luer taper of the catheter hub 102 and a male Luer taper of the male tip 220 register to form a Luer engagement and block distal advancement of the male tip further into the opening of the catheter hub. A seal can be provided by the Luer engagement to prevent fluid from leaking out the proximal opening 121 of the catheter hub 102.

As the valve opener 134 moves distally by the distal advancement of the male tip 220, the head 150, or nose end, of the valve opener 134 is urged distally and pushes against the proximally facing surface of the valve 136. In particular, the distal end of the valve opener 134 initially pushes against the proximally facing surface of the valve 136. As the valve 136 is fixed inside the catheter hub 102, the flaps of the valve 136 are urged distally and radially by the valve opener 134, which is urged distally by the male tip 220. For example, the head 150 contacts and pushes the valve 136 in the distal direction thereby opening a flow path 226 through the valve opening 325 of the valve 136. Fluid from the male tip 220 can then flow through the catheter hub 102, through the valve 136, and through the lumen of the catheter tube 104. Alternatively, a suction can be applied by the male medical instrument, such as a syringe or vacuum blood collection tube, and blood aspirated from the patient. This is often done for testing samples before infusion therapy is commenced. Also, typically any remaining blood is first flushed from the inside of the catheter hub 102 before infusion therapy is commenced.

With reference now to FIGs. 10A-10D, an end view, an isometric view, a top open view, and a top closed view, respectively, of a valve actuator or valve opener 134 in accordance with additional aspects of the present disclosure are shown, prior to placement inside a catheter hub 102. The valve opener 134 of the present embodiment can comprise an actuator head 150, sometimes referred to its abbreviated form as head, nose section or nose tip, comprising a through opening 164 and a pair of valve opener arms 152, which may also be referred to as leg elements or elongated extensions, extending in a proximal direction, either directly or indirectly, from the head 150. The head or nose section is arranged at the distal end of the valve opener and is configured to be pushed into the valve to open the valve. The through opening 164 allows the needle to pass therethrough during assembly in a ready to use position and for fluid to flow through during fluid administration following successful venipuncture or intravenous access.

The valve opener 134 of the present embodiment shares similar aspects with the valve opener 134 described elsewhere herein with a few exceptions. In the present embodiment, the actuator head 150 has a generally cylindrical shaped body 158 with an optional radiused distal end 270 and the two valve opener arms 152. Each opener arm 152 has a proximal end 151 and the two proximal ends 151 are spaced from one another a first distance in a first position of the two opener arms and are pressed or moved together closer to one another a second distance in a second position of the two opener arms, and wherein the second distance is smaller than the first distance, as further discussed below. The terms first, second, etc., are merely place holders or identifiers and serve to merely distinguish two different positions, unless the context indicates otherwise. Said differently, the valve opener 134 of the present embodiment can be fabricated with valve opener arms 152 that are spread apart at their respective proximal ends 151 to facilitate coupling or mounting with a needle guard and that can then move together to close over the needle guard. This configuration of the valve opener 134 minimizes compression or distortion of the needle guard during assembly as the spaced apart valve opener arms 152 function as an enlarged entrance or opening for coupling the needle guard and the valve opener together during assembly.

In the present embodiment, the actuator head 150 is shaped for one-time actuation when advanced by a male Luer tip following successful venipuncture to open the valve 136. For example, the body 158 of the actuator head 150 can project through the one or more slits of the valve 136 and remain attached to the valve even after the male Luer tip is removed. In other examples, the valve 136 can be configured with sufficient elastic force and/or the body 158 of the actuator head 150 is sized with a greater taper for the valve flaps to impart component forces to move the valve actuator in a proximal direction after removal of the male Luer tip, for a multi-use valve system. Optionally or additionally, a helical extension spring or an elastic gasket may be used to provide an external axial force to assist the valve actuator 134 to move away from the valve and in the proximal direction after removal of the male Luer tip from the proximal end of the catheter hub.

The present valve opener 134 is fabricated with the two valve opener arms 152 radially spaced from one another, when viewing the arms' respective proximal ends 151. In this spaced configuration, as shown in FIGs. 10A-10C, an enlarged gap 154 is defined by or is provided between the two spaced apart actuator arms or valve opener arms 152 in the arms' first position. However, when the two arms 152 are moved together in the arms' second position, as shown in FIG. 10D, the gap 154 between the two arms 152 is decreased. Thus, the present valve actuator 134 has a gap 154 between the two valve opener arms 152 having a first dimension, in which the two arms 152 are in their respective first positions, and a gap between the two valve opener arms having a second dimension, in which the two arms are in the respective second positions. The gap 154 in the second position with the second dimension is smaller than the first dimension, and can be as little as zero. The larger first dimension of the gap 154, due to the two valve opener arms 152 being spaced from one another at their respective proximal ends 151, provides ample space for coupling the valve opener 134 with a needle guard or for mounting the needle guard with the valve opener, as further discussed below. Thus, the valve opener 134 is configured to facilitate assembly by providing an enlarged gap 154, or opening, in a first configuration of the valve opener that can then be reduced in a second configuration of the valve opener after pairing the valve opener 134 with the needle guard 132 (FIG. 4). This arrangement allows for the assembly of the valve actuator and needle guard to be performed while minimizing bending and/or compressing the needle guard to get the arms 288, 290 of the needle guard, and the distal walls 300, 302 (FIG. 4) of the needle guard, through the opening 278 defined by the two actuator arms 152 and the two bridges 155 of the valve actuator 134, which can damage the needle guard.

As shown in FIG. 10D, two bridges or stabilizer elements 155 are incorporated connecting to the two valve opener arms 152. When in the configuration shown, the valve opener 134 has a distal cavity opening or chamber 386 and a proximal cavity opening or chamber 387. As shown in FIGs. 10A-10C, each of the two bridges 155 is formed from partial bridges or partial stabilizer elements 155a, 155b. In particular, each of the two actuator arms 152 comprises a pair of partial bridges or stabilizer elements 155a, 155b. When the two valve opener arms 152 are moved closer together in their respective second positions, the four partial bridges or partial stabilizer elements 155a, 155b form two bridges or stabilizer elements 155 each with a seam 272. In an example, two adjacent partial bridges 155a, 155b can be secured to one another after they contact to form a bridge 155, such as by bonding, gluing, or welding. In still other examples, the partial bridges can be provided with detents along the inner edges 155c for snapping together in mechanical engagement, and optionally with bonding gluing or welding. In another example, the adjacent partial bridges 155a, 155b are not secured to one another after they contact to form a bridge 155 but are constrained by the interior surface 289 of the catheter hub when the valve actuator 134 is placed inside the catheter hub, as further discussed below. When the arms 152 are moved together as shown in FIG. 10D, an opening 278 is defined by the two bridges 155 and the two actuator arms 152. To move an object from the proximal cavity opening or chamber 387 to the distal cavity opening or chamber 386 between the two bridges 155 in the configuration shown in FIG. 10D, the object needs to travel through the opening 278. Consequently, an object having a profile that is larger than the largest dimension of the opening 278 defined by the two bridges 155 and the two actuator arms 152 must compress or be distorted to be smaller than the opening 278 in order to pass therethrough. Therefore, by providing a valve opener with stabilizer elements 155 having a first and a second position, the object can be coupled to the valve opener without any or excessive distortion.

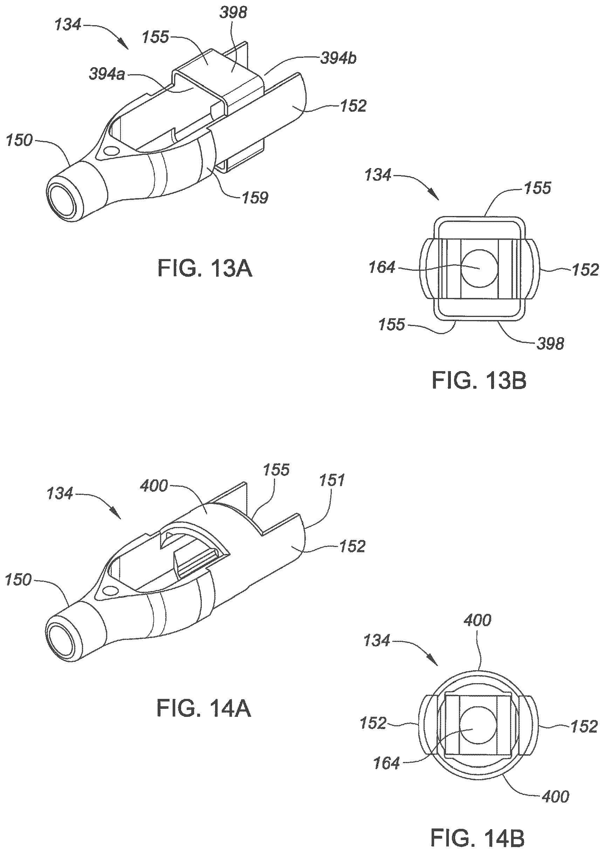

With continued reference to FIG. 10D, each bridge or stabilizer element 155 comprises a distal edge 394a and a proximal edge 394b. In an example, the distal and proximal edges 394a, 394b of each respective bridge 155 are parallel to one another. Relative to the lengthwise axis of the valve actuator 134, the distal and proximal edges 394a, 394b can be orthogonal or be angled to the lengthwise axis. Further and comparing to the edges of the opposing or other bridge, the edges can have different angles from one another, as shown in FIG. 10D, or be parallel to one another, as shown in FIGs. 13A-14B.

The distal edges 394a of the two stabilizer elements 155 provide structural surfaces for the intersection or elbow 304 between the distal wall 300 and the resilient arm 288 of the two arms 288, 290 to engage or abut to prevent the needle guard from moving in the proximal direction in a ready to use position and during retraction of the needle following successful venipuncture. Thus, the two stabilizer elements or bridges 155 retain the needle guard to the valve actuator 134 in both the ready position and during retraction of the needle following successful venipuncture. By engaging the needle guard to the two bridges 155, and more particularly abutting the two distal edges 394a of the two bridges, or by arranging the two bridges 155 to block proximal movement of the needle guard, the needle guard 134 does not need to engage the guard engagement section 210 inside the catheter hub 102 as previously described, such as with reference to FIG. 7 and elsewhere. However, when the two arms 288, 290 of the needle guard 132 are no longer biased outwardly by the needle, the diagonal dimension at the two elbows 304 of the needle guard decreases to a dimension that is smaller than the opening size at the two bridges 155 to then allow the needle guard to move proximally of the two bridges 155, and not be impeded by the distal edges of the two bridges.

The valve actuator 134 may be made from plastic injection molding and the two actuator arms 152 and partial bridges 155a, 155b formed in their first spaced apart positions as shown in FIGs. 10A-10C. Thus, in moving the arms together as shown in FIG. 10D, the arms 152 are held under strain and will tend to spring back open to its relaxed state shown in FIGs. 10A-10C. In an example, the two arms can be secured in the position shown in FIG. 10D by bonding or gluing the two sets of partial bridges 155a, 155b together. Alternatively, the two arms can be held closer together by placing the valve actuator inside a catheter hub and the interior surface of the catheter hub constraining the two arms. Depending on the clearance between the interior of the catheter hub and the valve actuator 134, the two arms 152 of the valve actuator 134 may naturally move away from one another inside the catheter hub up to the constraint of the catheter hub. Consequently, when constrained only by the catheter hub, the seams 272 at the two bridges 155 can widen to more than a line.

Exteriorly, guide tabs 159 can be provided on each of the two actuator arms 152. The guide tabs 159 can cooperate with surface features inside the catheter hub for alignment purposes and to retain the valve actuator inside the catheter hub. For example, the two tabs 159 can cooperate with a pair of slots 135 (FIG. 7) in the interior of a catheter hub to prevent the valve actuator from rotating about the lengthwise axis of the catheter hub. The pair of slots can also retain the valve actuator from displacing proximally out the proximal opening of the catheter hub or optionally different surface structures inside the catheter hub can be implemented to retain the valve actuator from displacing proximally out the proximal opening of the catheter hub.

With reference now to FIG. 10E, a valve opener 134 and a needle guard 132 are shown mounted on a needle 108 and the needle attached to a needle hub 106. When the needle guard 132 is mounted onto the needle, the needle biases the two arms of the needle guard outwardly. Where the needle guard incorporates a single arm, the single arm is biased by the needle. The valve opener 134 can be the same as shown in FIGs. 10A-10C and the needle guard can be the same as shown and described elsewhere, such as FIGs. 2-4. The configuration shown in FIG. 10E can be carried out by first securing the needle to the needle hub and then placing the needle guard onto the needle before adding the change in profile 144 near the needle tip 110 of the needle, such as by crimping to form a crimp. Alternatively, the needle guard can first be placed on the needle and then the needle attached to the needle hub prior to forming the change in profile, such as by crimping.

Next, the valve opener 134 can slide onto to the needle with the two spaced apart actuator arms 152 moved directly over the needle guard and ensuring that the elbows 304 of the two arms are distal of the partial bridges 155a, 155b when the arms 152 are moved closer together to their respective second positions. The through opening 164 at the body 158 of the actuator head 150 is sufficiently wide or large to readily slide onto the needle 108 and over the change in profile 144 without any interference. The partially assembled components shown in FIG. 10E may be referred to as a needle, guard, and actuator sub-assembly 380, or an NGA sub-assembly 380, which can also be referred to its abbreviated name as simply a sub-assembly. The sub-assembly is so formed prior to assembling with a catheter hub having a catheter tube, and prior to placing the valve opener into the interior of the catheter hub.

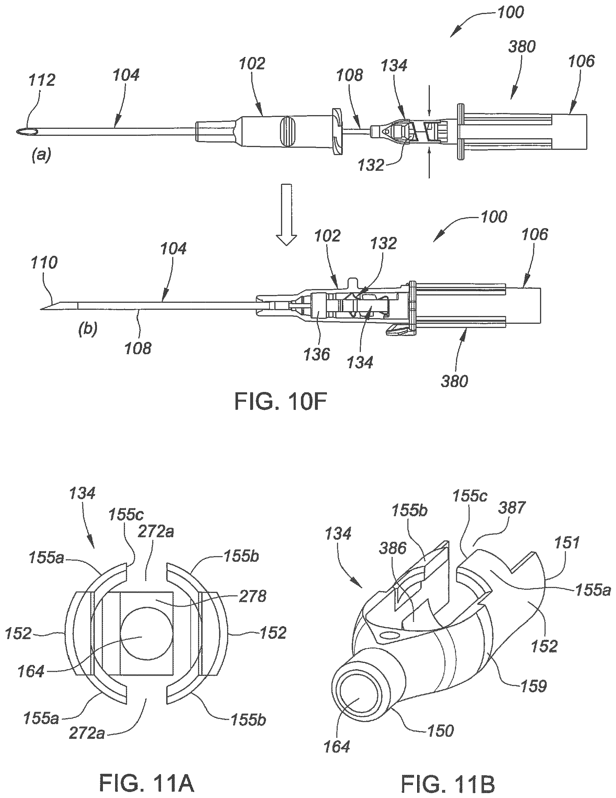

With reference now to FIGs. 10F (a) and (b), the NGA sub-assembly 380 is shown partially inserted into a catheter hub 102 having a catheter tube 104 attached thereto (in FIG. 10F(a)). During insertion of the NGA sub-assembly 380, the two actuator arms 152 are held together in their respective second positions to provide clearance for insertion of the valve actuator 134 into the proximal opening of the catheter hub. As previously described, the two actuator arms can be held together by gluing or bonding the partial stabilizer elements. In other examples, detents may be use to engage the partial stabilizer elements.

FIG. 10F(b) shows the NGA sub-assembly 380 fully inserted into the catheter hub 102 to form a catheter assembly 100 having the valve actuator 134 of the present embodiment and a needle guard 132 secured inside a catheter hub 102 without having to compress or deform the two arms of the needle guard to assemble the needle guard to the valve actuator. The valve actuator 134 is shown located proximally of a valve 136. In the example shown, the valve can have a skirt, similar to the flange shown in FIG. 8A but with an extended length, defining a cavity and the actuator head of the valve opener located in the cavity of the skirt. In alternative embodiments, the valve can be without a skirt, similar to the valve disc shown in FIGs. 8A-9B. The nose section of the needle hub is shown located in the proximal opening of the catheter hub. In an example, the nose section can be used to push the proximal ends 152 of the valve opener into position inside the catheter hub. The present valve actuator may be used for infusion and with catheter hubs described elsewhere herein and in a similar manner as the valve opener described with reference to FIGs. 5-6.

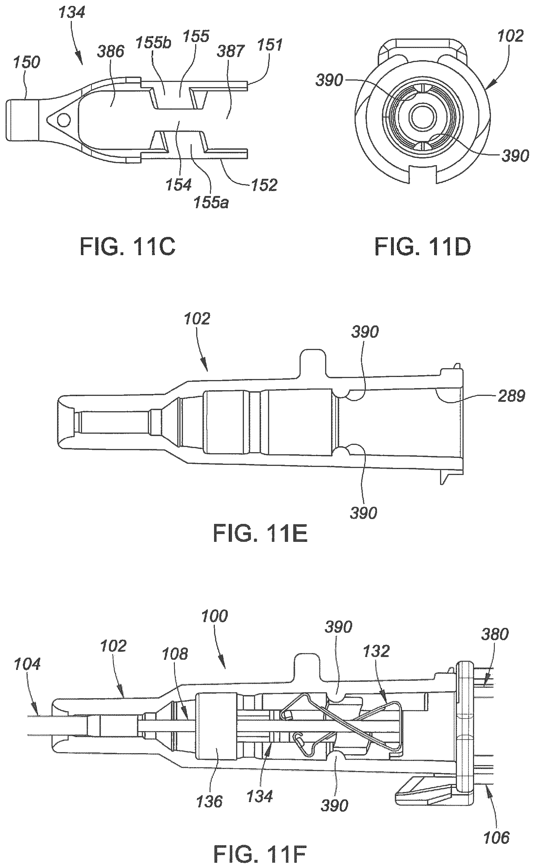

FIGs. 11A-11C show an end view, an isometric view, and a top view, respectively, of a valve actuator or valve opener 134 in accordance with further aspects of the present disclosure, which can be used with catheter assemblies described elsewhere herein and in a similar manner as the valve opener described with reference to FIGs. 5-6. The valve opener 134 shares similar aspects with the valve opener 134 of FIGs. 10A-10D with a few exceptions. In the present embodiment, the two actuator arms 152 are fabricated in a normal extended position from the actuator head 150, and not spaced apart as shown in FIGs. 10A-10C. Further, the partial stabilizer elements 155a, 155b are shorter along the radial direction compared to the partial stabilizer elements of FIGs. 10A-10D. Thus, the actuator arms 152 are not intended to deflect radially outwardly or radially inwardly relative to a lengthwise axis of the valve actuator although the arms 152 can deflect since the partial stabilizer elements 155a, 155b are not configured to abut or contact along their inner edges 155c and are provided with an enlarged seams 272a and a gap. Consequently, because of the enlarged seams 272a, clearance is provided for the arms 152 to deflect radially outwardly or inwardly relative to a lengthwise axis of the valve actuator 134 without the inner edges 155c delimiting the radially inward deflection.

In an example, each of the two enlarged seams 272a of the two pairs of partial bridges 155a, 155b has a width or a gap 154. The width or gap can be measured between the adjacent inner edges 155c of two adjacent partial bridges 155a, 155b. In an example, the dimension of the width or gap 154 of each enlarged seam 272a is smaller or narrower than a width of a distal wall 300, 302 of the needle guard 132, or at least smaller than the dimension of the arms 288, 290 of the needle guard at the elbows 304 (FIG. 4). This arrangement allows the two bridges 155 of the present valve actuator or opener 134 to impede proximal movement of the needle guard 132 in the ready position and during retraction of the needle following successful venipuncture, even with the enlarged seams 272a.

To couple the needle guard 132 to the valve actuator 134 of the present embodiment so that the two elbows 304 of the needle guard are located distal of the distal edges of the partial stabilizers 155a, 155b, at the distal cavity opening or chamber 386 of the valve actuator 134, without compressing or bending the needle guard, the two actuator arms 152 can be deflected outwardly relative to the longitudinal axis of the valve actuator 134 from the first position shown in FIGs. 11A-11C to a second position in which the proximal ends 151 are spaced apart further than as shown to widen the enlarged seams 272a of the two pairs of partial bridges. The now further widened enlarged seams 272a allow the valve actuator 134 to be placed over the needle guard 132, while the needle guard is mounted on a needle, without deflecting or compressing the needle guard to fit through the opening 278 defined by the two actuator arms 152 and the four partial bridges 155a, 155b. The two actuator arms 152 can then relax and move closer together.

FIGs. 11D and 11E show an end view and side-cross sectional view of a catheter hub 102 provided in accordance with aspects of the present invention for use with the valve actuator 134 of FIGs. 11A-11C. As shown, the interior surface 288 of the catheter hub is provided with a couple of bumps or projections 390, which are located and oriented inside the catheter hub to align with and occupy the spaces at the two enlarged seams 272a when the valve actuator is located inside the catheter hub during assembly.

As shown, the two projections 390 are equally spaced within the interior of the catheter hub. When located between or inside the seams 272a, the two projections 390 are provided to prevent rotation of the valve actuator about the lengthwise axis of the catheter hub. The two projections 390 are also provided to limit deflection of the two arms 152 inwardly towards one another by occupying most if not all of the spaces defined by the two enlarged seams 272a. For example, a male Luer tip inserted into the catheter hub to advance the valve actuator 134 in the distal direction to open a valve can cause the two actuator arms 152 to deflect inwardly towards one another if not for the projections. Thus, the projections operate to limit the inward deflection. In an alternative embodiment, only one projection is incorporated inside the catheter hub.

During assembly, an NGA sub-assembly may first be formed for the present embodiment. The NGA sub-assembly can include a needle, a guard, and an actuator, similar to that shown with FIG. 10E. The needle can be attached to a needle hub. The NGA sub-assembly with the valve opener of FIGs. 11A-11C and a needle guard, such as the needle guard 132 of FIG. 4, can be formed prior to assembling the sub-assembly to a catheter hub, such as prior to sliding the needle with the valve opener and needle guard into the catheter hub and the catheter tube.

With reference now to FIG. 11F, the NGA sub-assembly can be installed or assembled to the catheter hub and catheter tube as shown in FIG. 11F, which shows the valve actuator 134 located proximally of a valve 136 and the actuator head in a space defined by a shroud or skirt of the valve. In other examples, the valve can be without a shroud or a skirt. In the installed configuration, the two projections 390 are shown located proximally of the two elbows 304 of the needle guard, which is located between the two actuator arms 152. The two projections 390 generally align with the distal edges of the four partial bridges 155a, 155b and are located between the partial bridges.

As shown in FIG. 11F, the two projections 390 define a minimum inside diameter. Thus, as the NGA sub-assembly is inserted into the catheter hub, the two arms 288, 290 of the needle guard can undergo some deflection at the elbows 304 in order to fit within the minimum inside diameter at the two projections 390. However, assembly of the needle guard to the valve opener when forming the NGA sub-assembly can be carried out without compressing the two arms to the same extent, or any, to pass through the opening defined by the bridges and valve opener arms.

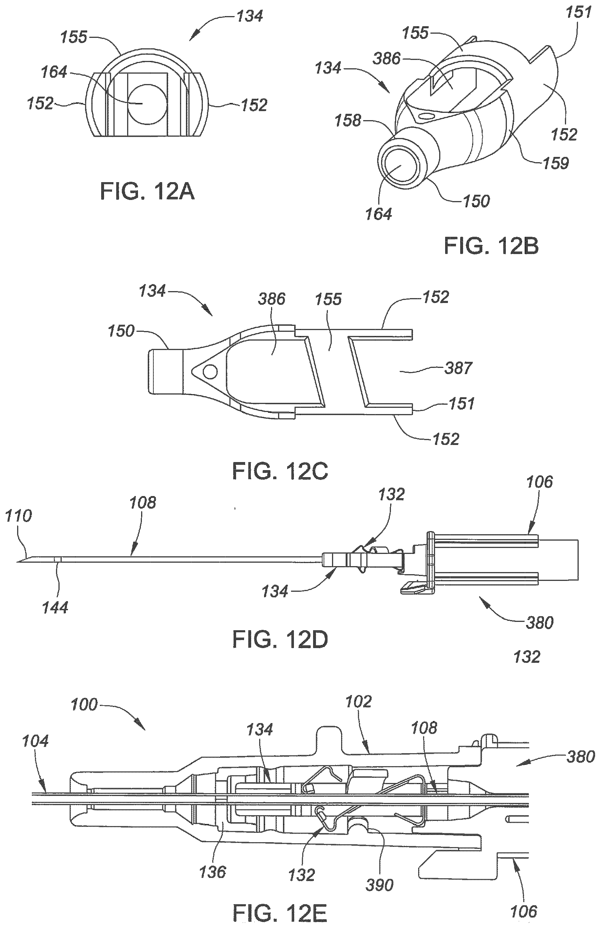

FIGs. 12A-12C show an end view, an isometric view, and a top view, respectively, of a valve actuator or valve opener 134 in accordance with further aspects of the present disclosure, which can be used with catheter assemblies described elsewhere herein and in a similar manner as the valve opener described with reference to FIGs. 5-6. The valve opener 134 of the present embodiment shares similar aspects with the valve opener 134 of FIGs. 10A-10D and the valve opener of FIGs. 11A-11C with a few exceptions. In the present embodiment, the two actuator arms 152 are fabricated in a normal extended position from the actuator head 150 and are connected to one another with a single bridge or stabilizer element 155. The single bridge 155 does not have a seam and does not readily allow the two arms 152 to deflect inwardly or outwardly relatively to the lengthwise axis of the valve actuator 134.

With specific reference to FIG. 12A, as the present valve actuator 134 only incorporates a single stabilizer element 155, the present valve actuator does not have a restricted or defined opening defined by two actuator legs 152 and two stabilizer elements 155. Said differently and as clearly shown in FIG. 12A, the valve actuator 134 of the present embodiment does not have a fully enclosed structure along the radial direction, and can be described as roughly U-shaped. Accordingly, when the valve actuator 134 of the present embodiment is coupled to a needle guard 132 (FIG. 4) to form an NGA sub-assembly, the valve actuator can be manipulated back-and-forth and up-and-down to move over the needle guard without compressing or distorting the needle guard to do so, or only some minimum amount to do so, due at least to the open bottom of the valve actuator 134 not incorporating a second bridge or stabilizer element.

With reference to FIG. 12D, an NGA sub-assembly 380 is shown, which comprises a needle, a guard, and an actuator. The needle is shown attached to a needle hub. The valve actuator 134 of FIGs. 12A-12C is shown located over the needle guard 132, which can be assembled without distorting or compressing the needle guard, or only some minimum amount to do so, due at least in part to the open bottom of the valve actuator, without a second stabilizer element. The NGA sub-assembly 380 of the present embodiment can be formed prior to inserting the needle into the catheter hub and needle hub, and prior to placement of the valve opener into the catheter hub.

FIG. 12E shows the NGA sub-assembly 380 of FIG. 12D inserted into and assembled to the catheter hub 102 and catheter tube 104, which shows the valve actuator 134 located proximally of a valve 136. Unlike the catheter hub of FIGs. 11D-11F, the present catheter hub 102 incorporates a single projection 390. In the installed configuration of FIG. 12E, the single projection 390 is shown located proximally of the one of the two elbows 304 of the needle guard, on the side without the stabilizer element or bridge 155. The location of the single bridge 155, which has a distal edge and a proximal edge, and the location of the single projection 390 can be on opposite sides of the lengthwise axis of the catheter hub. The projection 390 is generally aligned with the distal edge of the bridge 155, and is proximally of the elbow 304 of the needle guard. In the configuration shown, the stabilizer element or bridge 155 and the projection 390 both prevent the needle guard 132 from displacing in the proximal direction in the ready position and during retraction of the needle following successful venipuncture.

As shown in FIG. 12E, the single projection 390 and the single bridge 155 define a minimum inside diameter in the catheter hub. Thus, as the NGA sub-assembly is inserted into the catheter hub, the two arms 288, 290 of the needle guard can undergo some deflection at the elbows 304 in order to fit within the minimum inside diameter at the projection 390 and the bridge 155.

FIGs. 13A-13B show an isometric view and an end view, respectively, of a valve actuator or valve opener 134 in accordance with further aspects of the present disclosure, which can be used with catheter assemblies described elsewhere herein and in a similar manner as the valve opener described with reference to FIGs. 5-6. The valve opener 134 of the present embodiment shares similar aspects with the valve opener 134 of FIGs. 10A-10D and the valve opener of FIGs. 11A-11C with a few exceptions. In the present embodiment, the two stabilizer elements 155 connect the two actuator arms 152 together and they do not incorporate any seam. Further, the contour of the two stabilizer elements 155 are generally flat or planar. Said differently, each stabilizer element comprises a planar surface 398 rather than an arcuate contour. Further, the distal edge 394a and the proximal edge 394b of each stabilizer element 155 are generally parallel to one another and to the distal and proximal edges 394a, 394b of the other stabilizer element, or second stabilizer element. In use, the valve actuator 134 of the present embodiment can be assembled or coupled with a needle guard and then the combination mounted onto a needle before a change in profile is formed with the needle. Thus, an NGA sub-assembly can be formed with the present valve actuator prior to placing the valve actuator inside a catheter hub. Optionally, the present valve actuator can be placed inside a catheter hub prior to positioning a needle with a needle guard into the catheter hub and using the nose section of the needle hub to push the needle guard in through the opening defined by the two stabilizer elements and the two valve opener arms.

FIGs. 14A-14B show an isometric view and an end view, respectively, of a valve actuator or valve opener 134 in accordance with still further aspects of the present disclosure, which can be used with catheter assemblies described elsewhere herein and in a similar manner as the valve opener described with reference to FIGs. 5-6. The valve opener 134 of the present embodiment shares similar aspects with the valve opener 134 of FIGs. 13A-13B with a few exceptions. In the present embodiment, the two stabilizer elements 155 connect the two actuator arms 152 together and they do not incorporate any seam. Further, the contour of the two stabilizer elements 155 are generally arcuate rather than planar, as shown in FIGs. 13A-13B. Said differently, each stabilizer element 155 of the present embodiment comprises an arcuate or curved surface 400 that curves away from the lengthwise axis of the valve opener. Further, the distal edge 394a and the proximal edge 394b of each stabilizer element 155 are generally parallel to one another and to the distal and proximal edges 394a, 394b of the other stabilizer element. In use, the valve actuator 134 of the present embodiment can be assembled or coupled with a needle guard and then the combination mounted onto a needle before a change in profile is formed with the needle. Thus, an NGA sub-assembly can be formed with the present valve actuator prior to placing the valve actuator inside a catheter hub. Optionally, the present valve actuator can be placed inside a catheter hub prior to positioning a needle with a needle guard into the catheter hub and using the nose section of the needle hub to push the needle guard in through the opening defined by the two stabilizer elements and the two valve opener arms.

Methods of making the catheter assemblies and their components described elsewhere herein are within the scope of the present disclosure.

Although limited embodiments of catheter assemblies and their components have been specifically described and illustrated herein, many modifications and variations will be apparent to those skilled in the art. For example the needle guard may be of one piece or can be integrated from more than one piece, such as from multiple pieces that can be all metal or a combination of metal and polymer materials, such as plastic. Furthermore, it is understood and contemplated that features specifically discussed for one catheter assembly or for one component may be adopted for inclusion with another catheter assembly or another component, provided the functions are compatible. Accordingly, it is to be understood that the catheter assemblies and their components constructed according to principles of the disclosed devices, systems, and methods may be embodied other than as specifically described herein. The valves and valve openers described herein can also be used with a needle hub by locating them inside a female Luer taper of the needle hub. The valves and valve openers can also be used in the female connector of an infusion needle or a blood collection device or a central venous catheter or peripherally inserted central catheter (PICC). In other words, the valves and valve openers can be used in any medical device intended for infusion or bodily fluid collection with a female Luer housing or hub. The invention is defined in the following claims.

## Claims

1. A sub-assembly (380) for a needle assembly (100), said sub-assembly (380) comprising:
a valve actuator (134) comprising an actuator head (150) comprising a body (158) having a through opening (164), two valve opener arms (152) extending proximally of the body (158), and at least one bridge (155) connected to the two valve opener arms (152), said at least one bridge comprising a distal edge (394a) and a proximal edge (394b);
a needle guard (132) comprising a proximal wall (280) having a perimeter (282) defining a proximal opening (284), two arms (288, 290) extending distally of the proximal wall (280), and an elbow (304) on each of the two arms (288, 290); and
a needle (108) attached to a needle hub (106), said needle (108) extending through the needle guard (132) and the valve actuator (134) and comprises a change in profile (144) located proximally of a needle tip (110);
wherein the bridge (155) comprises a seam (272) or is continuously formed without a seam; and
wherein the elbow (304) of at least one of the two arms (288, 290) is positioned distally of the distal edge (394a) prior to placement of the needle (108) into a catheter hub (102).

2. The sub-assembly (380) of claim 1, further comprising a guide tab (159) on each of the two valve opener arms (152).

3. The sub-assembly (380) of claim 1 or 2, wherein the bridge (155) has the seam (272) and wherein each of the two valve opener arms (152) comprises a proximal end (151) and wherein the two proximal ends (151) are spaced from one another a first distance in a first position and spaced from none another a second distance in a second position.

4. The sub-assembly (380) of claim 3, wherein the first distance is greater than the second distance.

5. The sub-assembly (380) of claim 3, wherein the first distance is smaller than the second distance.

6. The sub-assembly (380) of any claim 1-5, further comprising a second bridge (155) connected to the two valve opener arms (152), wherein the second bridge (155) comprises a seam (272) or is continuously formed without a seam.

7. The sub-assembly (360) of any preceding claim, further comprising a catheter hub (102) and a catheter tube (104) and wherein the needle (108) projects through the catheter tube (104).

8. The sub-assembly (360) of claim 7, further comprising a valve (136) located in an interior of the catheter hub (102) along with the valve opener (134) and the needle guard (132).

9. The sub-assembly (360) of any preceding claim, wherein the bridge (155) has a distal edge (394a) and a proximal edge (394b) and wherein the distal edge (394a) is orthogonal to a lengthwise axis of the valve actuator (134) or angled to the lengthwise axis of the valve actuator (134).

10. The sub-assembly (360) of claim 9, wherein the distal edge (394a) and the proximal edge (394b) are parallel to one another.

11. A catheter assembly (100) comprising:
a catheter hub (102) having hub body (103) with an interior cavity (130) and a catheter tube (104) attached to the hub body (103);
a sub-assembly (380) connected to the catheter hub (102), said sub-assembly (380) comprising:
a valve actuator (134) comprising an actuator head (150) comprising a body (158) having a through opening (164), two valve opener arms (152) extending proximally of the body (158), and at least one bridge (155) connected to the two valve opener arms (152), said at least one bridge comprising a distal edge (394a) and a proximal edge (394b);
a needle guard (132) comprising a proximal wall (280) having a perimeter (282) defining a proximal opening (284), two arms (288, 290) extending distally of the proximal wall (280), and an elbow (304) on each of the two arms (288, 290); and
a needle (108) attached to a needle hub (106), said needle (108) extending through the needle guard (132), the valve actuator (134), and the catheter tube (104) and having a needle tip (110) extending distally of a distal opening (112) of the catheter tube (104), said needle (108) comprising a change in profile (144) located proximally of the needle tip (110);
wherein the bridge (155) comprises a seam (272) or is continuously formed without a seam; and
wherein the elbow (304) of at least one of the two arms (288, 290) is positioned distally of the distal edge (394a).

12. The catheter assembly (100) of claim 11, further comprising a valve (136) located in the interior cavity of the catheter hub (102).

13. The catheter assembly (100) of claim 11 or 12, wherein a slot (135) is formed in the interior cavity of the catheter hub (102) distal of a guard engagement section (210).

14. The catheter assembly (100) of claim 11 or 12, wherein a projection (390) is formed in the interior cavity of the catheter hub (102) and the seam (272) of the bridge (155) is configured to pass through the projection (390).

15. The catheter assembly (100) of claim 12, wherein the valve (136) comprises a flange (323a) defining a circular cavity (328a).

16. The catheter assembly (100) of claim 15, wherein the valve (136) comprises a plurality of slits 324 and a plurality of flaps (326).

17. The catheter assembly (100) of any claim 11-15, wherein the bridge (155) has a distal edge (394a) and a proximal edge (394b) and wherein the distal edge (394a) is orthogonal to a lengthwise axis of the valve actuator (134) or angled to the lengthwise axis of the valve actuator (134).

18. The catheter assembly (100) of claim 17, wherein the distal edge (394a) and the proximal edge (394b) are parallel to one another.

19. The catheter assembly (100) of claim 17 or 18, wherein an upper surface of the bridge (155) can have a flat planar surface or can have a curved surface.

20. A method of manufacturing a catheter assembly (100) comprising:
providing a catheter hub (102) having hub body (103) with an interior cavity (130) and a catheter tube (104) attached to the hub body (103);
connecting a sub-assembly (380) and the catheter hub (102) together, the sub-assembly (380) comprising:
a valve actuator (134) comprising an actuator head (150) comprising a body (158) having a through opening (164), two valve opener arms (152) extending proximally of the body (158), and at least one bridge (155) connected to the two valve opener arms (152), said at least one bridge comprising a distal edge (394a) and a proximal edge (394b);
a needle guard (132) comprising a proximal wall (280) having a perimeter (282) defining a proximal opening (284), two arms (288, 290) extending distally of the proximal wall (280), and an elbow (304) on each of the two arms (288, 290); and
a needle (108) attached to a needle hub (106), said needle (108) extending through the needle guard (132), the valve actuator (134), and the catheter tube (104) and having a needle tip (110) extending distally of a distal opening (112) of the catheter tube (104), said needle (108) comprising a change in profile (144) located proximally of the needle tip (110);
wherein the bridge (155) comprises a seam (272) or is continuously formed without a seam; and
wherein the elbow (304) of at least one of the two arms (288, 290) is positioned distally of the distal edge (394a).

## Patentansprüche

1. Unteranordnung (380) für eine Nadelanordnung (100), die Unteranordnung (380) aufweisend:
einen Ventilaktuator (134), einen Aktuatorkopf (150), aufweisend einen Körper (158), der eine Durchgangsöffnung (164) aufweist, zwei Ventilöffnerarme (152), die sich proximal des Körpers (158) erstrecken, und mindestens eine Brücke (155), die mit den zwei Ventilöffnerarmen (152) verbunden ist, die mindestens eine Brücke aufweisend eine distale Kante (394a) und eine proximale Kante (394b);
einen Nadelschutz (132), aufweisend eine proximale Wand (280), die einen Umfang (282) aufweist, der eine proximale Öffnung (284) definiert, zwei Arme (288, 290), die sich distal von der proximalen Wand (280) erstrecken, und einen Ellenbogen (304) an jedem der zwei Arme (288, 290); und
eine Nadel (108), die an einem Nadelansatz (106) angebracht ist, wobei sich die Nadel (108) durch den Nadelschutz (132) und den Ventilaktuator (134) erstreckt und eine Änderung eines Profils (144) aufweist, das sich proximal einer Nadelspitze (110) befindet;
wobei die Brücke (155) eine Naht (272) aufweist oder ohne eine Naht kontinuierlich ausgebildet ist; und
wobei der Ellenbogen (304) mindestens eines der zwei Arme (288, 290) vor einem Platzieren der Nadel (108) in einen Katheteransatz (102) distal der distalen Kante (394a) positioniert ist.

2. Unteranordnung (380) nach Anspruch 1, ferner aufweisend eine Führungslasche (159) an jedem der zwei Ventilöffnerarme (152).

3. Unteranordnung (380) nach Anspruch 1 oder 2, wobei die Brücke (155) die Naht (272) aufweist und wobei jeder der zwei Ventilöffnerarme (152) ein proximales Ende (151) aufweist und wobei die zwei proximalen Enden (151) um einen ersten Abstand in einer ersten Position voneinander beabstandet sind und um einen zweiten Abstand in einer zweiten Position voneinander beabstandet sind.

4. Unteranordnung (380) nach Anspruch 3, wobei der erste Abstand größer als der zweite Abstand ist.

5. Unteranordnung (380) nach Anspruch 3, wobei der erste Abstand kleiner als der zweite Abstand ist.

6. Unteranordnung (380) nach einem der Ansprüche 1 bis 5, ferner aufweisend eine zweite Brücke (155), die mit den zwei Ventilöffnerarmen (152) verbunden ist, wobei die zweite Brücke (155) eine Naht (272) aufweist oder ohne eine Naht kontinuierlich ausgebildet ist.

7. Unteranordnung (360) nach einem der vorstehenden Ansprüche, ferner aufweisend einen Katheteransatz (102) und einen Katheter (104) und wobei die Nadel (108) durch den Katheter (104) hindurch vorspringt.

8. Unteranordnung (360) nach Anspruch 7, ferner aufweisend ein Ventil (136), das sich in einem Inneren des Katheteransatzes (102) zusammen mit dem Ventilöffner (134) und dem Nadelschutz (132) befindet.

9. Unteranordnung (360) nach einem der vorstehenden Ansprüche, wobei die Brücke (155) eine distale Kante (394a) und eine proximale Kante (394b) aufweist und wobei die distale Kante (394a) orthogonal zu einer Längsachse des Ventilaktuators (134) oder zu der Längsachse des Ventilaktuators (134) abgewinkelt ist.

10. Unteranordnung (360) nach Anspruch 9, wobei die distale Kante (394a) und die proximale Kante (394b) parallel zueinander sind.

11. Katheteranordnung (100), aufweisend:
einen Katheteransatz (102), der einen Ansatzkörper (103) mit einem Innenhohlraum (130) und einen Katheter (104) aufweist, der an dem Ansatzkörper (103) angebracht ist;
eine Unteranordnung (380), die mit dem Katheteransatz (102) verbunden ist, die Unteranordnung (380) aufweisend:
einen Ventilaktuator (134), aufweisend einen Aktuatorkopf (150), aufweisend einen Körper (158), der eine Durchgangsöffnung (164) aufweist, zwei Ventilöffnerarme (152), die sich proximal des Körpers (158) erstrecken, und mindestens eine Brücke (155), die mit den zwei Ventilöffnerarmen (152) verbunden ist, die mindestens eine Brücke aufweisend eine distale Kante (394a) und eine proximale Kante (394b);
einen Nadelschutz (132), aufweisend eine proximale Wand (280), die einen Umfang (282) aufweist, der eine proximale Öffnung (284) definiert, zwei Arme (288, 290), die sich distal von der proximalen Wand (280) erstrecken, und einen Ellenbogen (304) an jedem der zwei Arme (288, 290); und
eine Nadel (108), die an einem Nadelansatz (106) angebracht ist, wobei sich die Nadel (108) durch den Nadelschutz (132), den Ventilaktuator (134) und den Katheter (104) erstreckt und eine Nadelspitze (110) aufweist, die sich distal von einer distalen Öffnung (112) des Katheters (104) erstreckt, die Nadel (108) aufweisend eine Änderung des Profils (144), das sich proximal der Nadelspitze (110) befindet;
wobei die Brücke (155) eine Naht (272) aufweist oder ohne eine Naht kontinuierlich ausgebildet ist; und
wobei der Ellenbogen (304) mindestens eines der zwei Arme (288, 290) distal der distalen Kante (394a) positioniert ist.

12. Katheteranordnung (100) nach Anspruch 11, ferner aufweisend ein Ventil (136), das sich in dem Innenhohlraum des Katheteransatzes (102) befindet.

13. Katheteranordnung (100) nach Anspruch 11 oder 12, wobei eine Kerbe (135) in dem Innenhohlraum des Katheteransatzes (102) distal von einem Schutzeingriffsabschnitt (210) ausgebildet ist.

14. Katheteranordnung (100) nach Anspruch 11 oder 12, wobei ein Vorsprung (390) in dem Innenhohlraum des Katheteransatzes (102) ausgebildet ist und die Naht (272) der Brücke (155) konfiguriert ist, um durch den Vorsprung (390) zu verlaufen.

15. Katheteranordnung (100) nach Anspruch 12, wobei das Ventil (136) einen Flansch (323a) umfasst, der einen kreisförmigen Hohlraum (328a) definiert.

16. Katheteranordnung (100) nach Anspruch 15, wobei das Ventil (136) eine Vielzahl von Schlitzen 324 und eine Vielzahl von Klappen (326) umfasst.

17. Katheteranordnung (100) nach einem der Ansprüche 11 bis 15, wobei die Brücke (155) eine distale Kante (394a) und eine proximale Kante (394b) aufweist und wobei die distale Kante (394a) orthogonal zu einer Längsachse des Ventilaktuators (134) oder zu der Längsachse des Ventilaktuators (134) abgewinkelt ist.

18. Katheteranordnung (100) nach Anspruch 17, wobei die distale Kante (394a) und die proximale Kante (394b) parallel zueinander sind.

19. Katheteranordnung (100) nach Anspruch 17 oder 18, wobei eine obere Oberfläche der Brücke (155) eine flache ebene Oberfläche aufweisen kann oder eine gekrümmte Oberfläche aufweisen kann.

20. Verfahren zum Herstellen einer Katheteranordnung (100), aufweisend:
Bereitstellen eines Katheteransatzes (102), der einen Ansatzkörper (103) mit einem Innenhohlraum (130) und einen Katheter (104) aufweist, der an dem Ansatzkörper (103) angebracht ist;
Verbinden einer Unteranordnung (380) und des Katheteransatzes (102) miteinander, die Unteranordnung (380) aufweisend:
einen Ventilaktuator (134), aufweisend einen Aktuatorkopf (150), aufweisend einen Körper (158), der eine Durchgangsöffnung (164) aufweist, zwei Ventilöffnerarme (152), die sich proximal des Körpers (158) erstrecken, und mindestens eine Brücke (155), die mit den zwei Ventilöffnerarmen (152) verbunden ist, die mindestens eine Brücke aufweisend eine distale Kante (394a) und eine proximale Kante (394b);
einen Nadelschutz (132), aufweisend eine proximale Wand (280), die einen Umfang (282) aufweist, der eine proximale Öffnung (284) definiert, zwei Arme (288, 290), die sich distal von der proximalen Wand (280) erstrecken, und einen Ellenbogen (304) an jedem der zwei Arme (288, 290); und
eine Nadel (108), die an einem Nadelansatz (106) angebracht ist, wobei sich die Nadel (108) durch den Nadelschutz (132), den Ventilaktuator (134) und den Katheter (104) erstreckt und eine Nadelspitze (110) aufweist, die sich distal von einer distalen Öffnung (112) des Katheters (104) erstreckt, die Nadel (108) aufweisend eine Änderung des Profils (144), das sich proximal der Nadelspitze (110) befindet;
wobei die Brücke (155) eine Naht (272) umfasst oder ohne eine Naht kontinuierlich ausgebildet ist; und
wobei der Ellenbogen (304) mindestens eines der zwei Arme (288, 290) distal der distalen Kante (394a) positioniert ist.

## Revendications

1. Sous-ensemble (380) destiné à un ensemble aiguille (100), ledit sous-ensemble (380) comprenant :
un actionneur de valve (134) comprenant une tête d'actionneur (150) comprenant un corps (158) ayant une ouverture traversante (164), deux bras d'ouverture de valve (152) s'étendant proximalement du corps (158), et au moins un pont (155) relié aux deux bras d'ouverture de valve (152), ledit au moins un pont comprenant un bord distal (394a) et un bord proximal (394b) ;
une protection d'aiguille (132) comprenant une paroi proximale (280) ayant un périmètre (282) définissant une ouverture proximale (284), deux bras (288, 290) s'étendant distalement de la paroi proximale (280), et un coude (304) sur chacun des deux bras (288, 290) ; et
une aiguille (108) fixée à une embase d'aiguille (106), ladite aiguille (108) s'étendant à travers la protection d'aiguille (132) et l'actionneur de valve (134) et comprenant un changement de profil (144) localisé proximalement d'une pointe d'aiguille (110) ;
dans lequel le pont (155) comprend une jointure (272) ou est formé de façon continue sans jointure ; et
dans lequel le coude (304) d'au moins l'un des deux bras (288, 290) est positionné distalement du bord distal (394a) avant mise en place de l'aiguille (108) dans une embase de cathéter (102).

2. Sous-ensemble (380) selon la revendication 1, comprenant en outre une languette de guidage (159) sur chacun des deux bras d'ouverture de valve (152).

3. Sous-ensemble (380) selon la revendication 1 ou 2, dans lequel le pont (155) a la jointure (272) et dans lequel chacun des deux bras d'ouverture de valve (152) comprend une extrémité proximale (151) et dans lequel les deux extrémités proximales (151) sont espacées l'une de l'autre d'une première distance dans une première position et espacées l'une de l'autre d'une seconde distance dans une seconde position.

4. Sous-ensemble (380) selon la revendication 3, dans lequel la première distance est supérieure à la seconde distance.

5. Sous-ensemble (380) selon la revendication 3, dans lequel la première distance est inférieure à la seconde distance.

6. Sous-ensemble (380) selon l'une quelconque revendication 1 à 5, comprenant en outre un second pont (155) relié aux deux bras d'ouverture de valve (152), dans lequel le second pont (155) comprend une jointure (272) ou est formé de façon continue sans jointure.

7. Sous-ensemble (360) selon l'une quelconque revendication précédente, comprenant en outre une embase de cathéter (102) et un tube de cathéter (104) et dans lequel l'aiguille (108) fait saillie à travers le tube de cathéter (104).

8. Sous-ensemble (360) selon la revendication 7, comprenant en outre une valve (136) localisée dans un intérieur de l'embase de cathéter (102) en même temps que le système d'ouverture de valve (134) et la protection d'aiguille (132).

9. Sous-ensemble (360) selon l'une quelconque revendication précédente, dans lequel le pont (155) a un bord distal (394a) et un bord proximal (394b) et dans lequel le bord distal (394a) est orthogonal à un axe longitudinal de l'actionneur de valve (134) ou incliné par rapport à l'axe longitudinal de l'actionneur de valve (134).

10. Sous-ensemble (360) selon la revendication 9, dans lequel le bord distal (394a) et le bord proximal (394b) sont parallèles l'un à l'autre.

11. Ensemble cathéter (100) comprenant :
une embase de cathéter (102) ayant un corps d'embase (103) avec une cavité intérieure (130) et un tube de cathéter (104) fixé au corps d'embase (103) ;
un sous-ensemble (380) relié à l'embase de cathéter (102), ledit sous-ensemble (380) comprenant :
un actionneur de valve (134) comprenant une tête d'actionneur (150) comprenant un corps (158) ayant une ouverture traversante (164), deux bras d'ouverture de valve (152) s'étendant proximalement du corps (158), et au moins un pont (155) relié aux deux bras d'ouverture de valve (152), ledit au moins un pont comprenant un bord distal (394a) et un bord proximal (394b) ;
une protection d'aiguille (132) comprenant une paroi proximale (280) ayant un périmètre (282) définissant une ouverture proximale (284), deux bras (288, 290) s'étendant distalement de la paroi proximale (280), et un coude (304) sur chacun des deux bras (288, 290) ; et
une aiguille (108) fixée à une embase d'aiguille (106), ladite aiguille (108) s'étendant à travers la protection d'aiguille (132), l'actionneur de valve (134) et le tube de cathéter (104) et ayant une pointe d'aiguille (110) s'étendant distalement de l'ouverture distale (112) du tube de cathéter (104), ladite aiguille (108) comprenant un changement de profil (144) localisé proximalement de la pointe d'aiguille (110) ;
dans lequel le pont (155) comprend une jointure (272) ou est formé de façon continue sans jointure ; et
dans lequel le coude (304) d'au moins l'un des deux bras (288, 290) est positionné distalement du bord distal (394a).

12. Ensemble cathéter (100) selon la revendication 11, comprenant en outre une valve (136) localisée dans la cavité intérieure de l'embase de cathéter (102).

13. Ensemble cathéter (100) selon la revendication 11 ou 12, dans lequel une encoche (135) est formée dans la cavité intérieure de l'embase de cathéter (102) distale d'une section de mise en prise de protection (210).

14. Ensemble cathéter (100) selon la revendication 11 ou 12, dans lequel une saillie (390) est formée dans la cavité intérieure de l'embase de cathéter (102) et la jointure (272) du pont (155) est conçue pour passer à travers la saillie (390).

15. Ensemble cathéter (100) selon la revendication 12, dans lequel la valve (136) comprend un rebord (323a) définissant une cavité circulaire (328a).

16. Ensemble cathéter (100) selon la revendication 15, dans lequel la valve (136) comprend une pluralité de fentes 324 et une pluralité de rabats (326).

17. Ensemble cathéter (100) selon l'une quelconque revendication 11 à 15, dans lequel le pont (155) a un bord distal (394a) et un bord proximal (394b) et dans lequel le bord distal (394a) est orthogonal à un axe longitudinal de l'actionneur de valve (134) ou incliné par rapport à l'axe longitudinal de l'actionneur de valve (134).

18. Ensemble cathéter (100) selon la revendication 17, dans lequel le bord distal (394a) et le bord proximal (394b) sont parallèles l'un à l'autre.

19. Ensemble cathéter (100) selon la revendication 17 ou 18, dans lequel une surface supérieure du pont (155) peut avoir une surface plane plate ou peut avoir une surface courbée.

20. Procédé de fabrication d'un ensemble cathéter (100) comprenant :
la fourniture d'une embase de cathéter (102) ayant un corps d'embase (103) avec une cavité intérieure (130) et un tube de cathéter (104) fixé au corps d'embase (103) ;
l'assemblage d'un sous-ensemble (380) et de l'embase de cathéter (102), le sous-ensemble (380) comprenant :
un actionneur de valve (134) comprenant une tête d'actionneur (150) comprenant un corps (158) ayant une ouverture traversante (164), deux bras d'ouverture de valve (152) s'étendant proximalement du corps (158), et au moins un pont (155) relié aux deux bras d'ouverture de valve (152), ledit au moins un pont comprenant un bord distal (394a) et un bord proximal (394b) ;
une protection d'aiguille (132) comprenant une paroi proximale (280) ayant un périmètre (282) définissant une ouverture proximale (284), deux bras (288, 290) s'étendant distalement de la paroi proximale (280), et un coude (304) sur chacun des deux bras (288, 290) ; et
une aiguille (108) fixée à une embase d'aiguille (106), ladite aiguille (108) s'étendant à travers la protection d'aiguille (132), l'actionneur de valve (134) et le tube de cathéter (104) et ayant une pointe d'aiguille (110) s'étendant distalement de l'ouverture distale (112) du tube de cathéter (104), ladite aiguille (108) comprenant un changement de profil (144) localisé proximalement de la pointe d'aiguille (110) ;
dans lequel le pont (155) comprend une jointure (272) ou est formé de façon continue sans jointure ; et
dans lequel le coude (304) d'au moins l'un des deux bras (288, 290) est positionné distalement du bord distal (394a).
